# EUROPÄISCHE PATENTSCHRIFT

(11) **EP 1 339 717 B1**
(45) Veröffentlichungstag und Bekanntmachung des Hinweises auf die Patenterteilung: **09.02.2005**
(21) Anmeldenummer: 01997488.0
(22) Anmeldetag: 09.11.2001
(51) Int. Cl.: C07D 471/04, A61K 31/437, A61P 9/00, A61P 15/00

(54) **NEUE CARBAMAT-SUBSTITUIERTE PYRAZOLOPYRIDINDERIVATE**
NOVEL CARBAMATE-SUBSTITUTED PYRAZOLOPYRIDINE DERIVATIVES
NOUVEAUX D RIV S DE PYRAZOLOPYRIDINE SUBSTITUTION CARBAMATE

(30) Priorität: 22.11.2000 DE 10057751
(43) Veröffentlichungstag der Anmeldung: 03.09.2003
(73) Patentinhaber: Bayer HealthCare AG, 51368 Leverkusen (DE)
(72) Erfinder: STASCH, Johannes-Peter, 42651 Solingen (DE); FEURER, Achim, 69259 Wilhelmsfeld (DE); WEIGAND, Stefan, 42115 Wuppertal (DE); STAHL, Elke, 51467 Bergisch Gladbach (DE); FLUBACHER, Dietmar, 79110 Freiburg (DE); ALONSO-ALIJA, Cristina, 42781 Haan (DE); WUNDER, Frank, 42117 Wuppertal (DE); LANG, Dieter, 42553 Velbert (DE); DEMBOWSKY, Klaus, Boston, MA 02116 (US); STRAUB, Alexander, 42117 Wuppertal (DE); PERZBORN, Elisabeth, 42327 Wuppertal (DE)
(86) Internationale Anmeldenummer: PCT/EP2001/012966
(87) Internationale Veröffentlichungsnummer: WO 2002/042300

(56) Entgegenhaltungen:
- WO-A-00/06568
- WO-A-00/06569

## Beschreibung

Die vorliegende Erfindung betrifft neue chemische Verbindungen, welche die lösliche Guanylatcyclase stimulieren, ihre Herstellung und ihre Verwendung als Arzneimittel, insbesondere als Arzneimittel zur Behandlung von Herz-Kreislauf-Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriposphat (GTP). Die bisher bekannten Vertreter dieser Familie lassen sich sowohl nach strukturellen Merkmalen als auch nach der Art der. Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch CO ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation . und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt führen kann.

Eine auf die Beeinflussung des cGMP-Signalweges in Organismen abzielende NOunabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisenzentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol (YC-1, Wu et al., Blood 84 (1994), 4226; Mülsch et al., Br.J.Pharmacol. 120 (1997), 681), Fettsäuren (Goldberg et al, J. Biol. Chem. 252 (1977), 1279), Diphenyliodonium-hexafluorophosphat (Pettibone et al., Eur. J. Pharmacol. 116 (1985), 307), Isoliquiritigenin (Yu et al., Brit. J. Pharmacol. 114 (1995), 1587) sowie verschiedene substituierte Pyrazolderivate (WO 98/16223).

Weiterhin sind in der WO 98/16507, WO 98/23619, WO 00/06567, WO 00/06568, WO 00/06569 und WO 00/21954 Pyrazolopyridinderivate als Stimulatoren der löslichen Guanylatcyclase beschrieben. In diesen Patentanmeldungen sind auch Pyrazolopyridine beschrieben, welche einen Pyrimidinrest in 3-Position aufweisen. Derartige Verbindungen weisen eine sehr hohe in vitro Aktivität bezüglich der Stimulation der löslichen Guanylatcyclase auf. Allerdings zeigte es sich, dass diese Verbindungen hinsichtlich ihrer in vivo-Eigenschaften wie beispielsweise ihrem Verhalten in der Leber, ihrem pharmakokinetischen Verhalten, ihrer Dosis-Wirkungsbeziehung oder ihrem Metabolisierungsweg einige Nachteile aufweisen.

Es war daher die Aufgabe der vorliegenden Erfindung, weitere Pyrazolopyridinderivate bereitzustellen, welche als Stimulatoren der löslichen Guanylatcyclase wirken, aber nicht die vorstehend aufgeführten Nachteile der Verbindungen aus dem Stand der Technik aufweisen.

Diese Aufgabe wird gemäß der vorliegenden Erfindungen durch Verbindungen gemäß Anspruch 1 gelöst. Diese neuen Pyrazolopyridinderivate zeichnen sich durch einen Pyrimidinrest in 3-Position aus, der ein bestimmtes Substitutionsmuster aufweist, nämlich einen Carbamat- oder Thiocarbamatrest in 5-Position des Pyrimidinrings sowie eine Aminogruppe oder eine Dialkylamidgruppe in 4-Position des Pyrimidinrings.

Im einzelnen betrifft die vorliegende Erfindung Verbindungen der Formel (I) 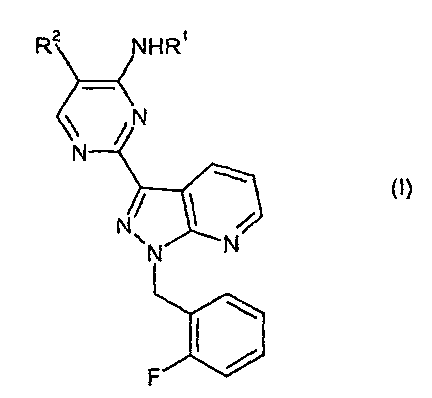 worin
- R¹: für Wasserstoff oder einen Di-C₁₋₆-alkylaminocarbonylrest steht,
- R²: für einen Rest der Formel -O-CX-NR³R⁴ steht,
wobei
X für O oder S steht;
R³ und R⁴ gleich oder voneinander verschieden sein können und für einen Rest aus der Gruppe, bestehend aus H, C₁₋₆-Alkyl, das gegebenenfalls einen CN- oder einen Halogensubstituenten trägt, C₁₋₆-Alkoxy-C₁₋₆-alkyl, Hydroxy-C₁₋₆-alkyl, C₂₋₆-Alkenyl, C₁₋₆-Alkylcarbonyloxy-C₁₋₆-alkyl, C₁₋₆-Alkoxycar-bonyl-C₁₋₆-alkyl, Hydroxycarbonyl-C₁₋₆-alkyl, Phenyl, p-Tolyl, einem über einen C₁₋₆-Alkandiylrest an das Stickstoff-atom gebundenen gesättigten fünf- bis siebengliedrigen Heterocyclus mit bis zu 2 Sauerstoffatomen oder C₃₋₈-Cycloalkyl, steht, wobei R³ und R⁴ nicht gleichzeitig H sein können; oder
R³ und R⁴ zusammen mit dem Stickstoffatom, an das sie gebunden sind, einen fünf- bis siebengliedrigen gesättigten Heterocyclus bilden, der gegebenenfalls ein weiteres Heteroatom aus der Gruppe N, O, S enthalten und/oder gegebenenfalls substituiert bzw. mit einem Phenylring anelliert sein kann;
sowie Salze, Isomere und Hydrate davon.

Bevorzugt sind hierbei Verbindungen der Formel (I), bei denen
- R¹: für Wasserstoff oder einen Diethylaminocarbonylrest steht,
- R²: für einen Rest der Formel -O-CX-NR³R⁴ steht,
wobei
X für O oder S steht;
R³ und R⁴ gleich oder voneinander verschieden sein können und für einen Rest aus der Gruppe, bestehend aus H, Methyl, Ethyl, iso-Propyl, Butan-2-yl, Methoxyethyl, 2-Methoxy-1-methylethyl, 1-Cyano-1-methylethyl, 2-Cyanoethyl, 2-Chlorethyl, Ethoxycarbonylmethyl, Hydroxycarbonylmethyl, 2-Propenyl, Phenyl, p-Tolyl, 1,3-Dioxolan-2-methyl, Cyclohexyl oder Cyclopentyl, steht, wobei R³ und R⁴ nicht gleichzeitig H sein können;
oder
R³ und R⁴ zusammen mit dem Stickstoffatom, an das sie gebunden sind, einen fünf- oder sechsgliedrigen gesättigten Heterocyclus bilden, der gegebenenfalls ein weiteres Heteroatom aus der Gruppe N, O enthalten und/oder gegebenenfalls einen Substituenten aus der Gruppe Methylcarbonyl, Ethoxycarbonyl oder t-Butoxycarbonyl, tragen kann, oder zusammen für 1,2,3,4Tetrahydrochinolin-N-yl stehen;
sowie Salze, Isomere und Hydrate davon.

Die erfindungsgemäßen Verbindungen der allgemeinen Formel (I) können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure,. Ethansulfonsäure, p-Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise, beispielsweise durch chromatographische Trennung, in die stereoisomer einheitlichen Bestandteile trennen. In den erfindungsgemäßen Verbindungen vorhandene Doppelbindungen können in der cis- oder trans- Konfiguration (Z- oder E-Form) vorliegen.

Weiterhin können bestimmte Verbindungen in tautomeren Formen vorliegen. Dies ist dem Fachmann bekannt, und derartige Verbindungen sind ebenfalls vom Umfang der Erfindung umfasst.

Weiterhin können die erfindungsgemäßen Verbindungen in Form ihrer Hydrate vorkommen, wobei die Zahl der an das Molekül gebundenen Wassermoleküle von der jeweiligen erfindungsgemäßen Verbindung abhängt.

Im Rahmen der vorliegenden Erfindung haben die Substituenten soweit nicht anders angegeben im allgemeinen die folgende Bedeutung:

Alkyl steht im allgemeinen für einen geradkettigen oder verzweigten Kohlenwasserstoffrest mit 1 bis 20 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl, Nonyl, Decyl, Dodeyl, Eicosyl genannt.

Alkylen steht im allgemeinen für eine geradkettige oder verzweigte Kohlenwasserstoffbrücke mit 1 bis 20 Kohlenstoffatomen. Beispielsweise seien Methylen, Ethylen, Propylen, α-Methylethylen, β-Methylethylen, α-Ethylethylen, β-Ethylethylen, Butylen, α-Methylpropylen, β-Methylpropylen, γ-Methylpropylen, α-Ethylpropylen, β-Ethylpropylen, γ-Ethylpropylen, Pentylen, Hexylen, Heptylen, Octylen, Nonylen, Decylen, Dodeylen und Eicosylen genannt.

Alkenyl steht im allgemeinen für einen geradkettigen oder verzweigten Kohlenwasserstoffrest mit 2 bis 20 Kohlenstoffatomen und einer oder mehreren, bevorzugt mit einer oder zwei Doppelbindungen. Beispielsweise seien Allyl, Propenyl, Isopropenyl, Butenyl, Isobutenyl, Pentenyl, Isopentenyl, Hexenyl, Isohexenyl, Heptenyl, Isoheptenyl, Octenyl, Isooctenyl genannt.

Acyl steht im allgemeinen für geradkettiges oder verzweigtes Niedrigalkyl mit 1 bis 9 Kohlenstoffatomen, das über eine Carbonylgruppe gebunden ist. Beispielsweise seien genannt: Acetyl, Ethylcarbonyl, Propylcarbonyl, Isopropylcarbonyl, Butylcarbonyl und Isobutylcarbonyl.

Alkoxy steht im allgemeinen für einen über einen Sauerstoffatom gebundenen geradkettigen oder verzweigten Kohlenwasserstoffrest mit 1 bis 14 Kohlenstoffatomen. Beispielsweise seien Methoxy, Ethoxy, Propoxy, Isopropoxy, Butoxy, Isobutoxy, Pentoxy Isopentoxy, Hexoxy, Isohexoxy, Heptoxy, Isoheptoxy, Octoxy oder Isooctoxy genannt. Die Begriffe "Alkoxy" und "Alkyloxy" werden synonym verwendet.

Alkoxyalkyl steht im allgemeinen für einen Alkylrest mit bis zu 8 Kohlenstoffatomen, der durch einen Alkoxyrest mit bis zu 8 Kohlenstoffatomen substituiert ist.

Alkoxycarbonyl kann beispielsweise durch die Formel 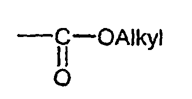 dargestellt werden.

Alkyl steht hierbei im allgemeinen für einen geradkettigen oder verzweigten Kohlenwasserstoffrest mit 1 bis 13 Kohlenstoffatomen. Beispielsweise seien die folgenden Alkoxycarbonylreste genannt: Methoxycarbonyl, Ethoxycarbonyl, Propoxycarbonyl, Isopropoxycarbonyl, Butoxycarbonyl oder Isobutoxycarbonyl

Cycloalkyl steht im allgemeinen für einen cyclischen Kohlenwasserstoffrest mit 3 bis 8 Kohlenstoffatomen. Bevorzugt sind Cyclopropyl, Cyclopentyl und Cyclohexyl. Beispielsweise seien Cyclopentyl, Cyclohexyl, Cycloheptyl und Cyclooctyl genannt.

Halogen steht im Rahmen der Erfindung für Fluor, Chlor, Brom und Iod.

Heterocyclus steht im Rahmen der Erfindung im allgemeinen für einen gesättigten, ungesättigten oder aromatischen 3- bis 10-gliedrigen, beispielsweise 5- oder 6-gliedrigen Heterocyclus, der bis zu 3 Heteroatome aus der Reihe S, N und/oder O enthalten kann und der im Fall eines Stickstoffatoms auch über dieses gebunden sein kann. Beispielsweise seien genannt: Oxadiazolyl, Thiadiazolyl, Pyrazolyl, Pyridyl, Pyrimidinyl, Pyridazinyl, Pyrazinyl, Thienyl, Furyl, Pyrrolyl, Pyrrolidinyl, Piperazinyl, Tetrahydropyranyl, Tetrahydrofuranyl, 1,2,3 Triazolyl, Thiazolyl, Oxazolyl, Imidazolyl, Morpholinyl oder Piperidyl. Bevorzugt sind Thiazolyl, Furyl, Oxazolyl, Pyrazolyl, Triazolyl, Pyridyl, Pyrimidinyl, Pyridazinyl und Tetrahydropyranyl. Der Begriff "Heteroaryl" (bzw. "Hetaryl") steht für einen aromatischen heterocyclischen Rest.

Die erfindungsgemäßen Verbindungen der Formel (I) können hergestellt werden durch die Umsetzung der Verbindung der Formel (II) 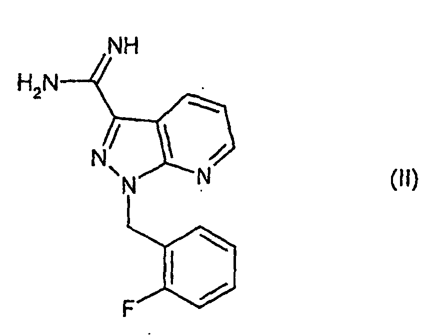 mit der Verbindung der Formel (III) 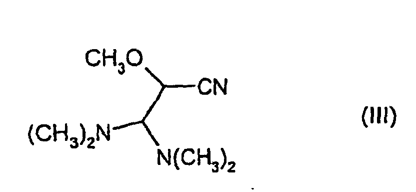 in einem organischen Lösungsmittel in Gegenwart einer Base unter Erhitzen und anschließender Überführung der Ethergruppe in die freie Hydroxygruppe zu Verbindungen der Formel (IV) 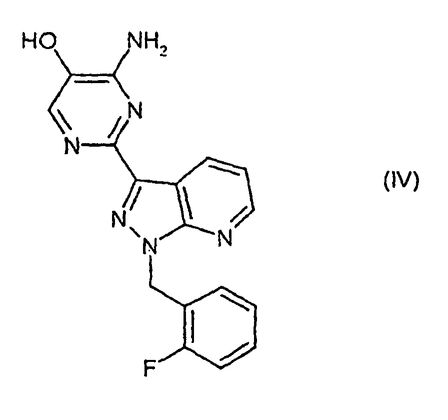 sowie die anschließende Umsetzung mit Verbindungen der Formel X-CO-NR³R⁴,
worin
- X: für einen Halogenrest oder Alkoxyrest steht,
- R³ und R⁴: die vorstehend angegebene Bedeutung haben;
in einem organischen Lösungsmittel gegebenenfalls in Gegenwart einer Base sowie gegebenenfalls unter anschließender Entfernung von Schutzgruppen zu Verbindungen der Formel (I).

Die Verbindung der Formel (II) lässt sich gemäß folgendem Reaktionsschema herstellen:

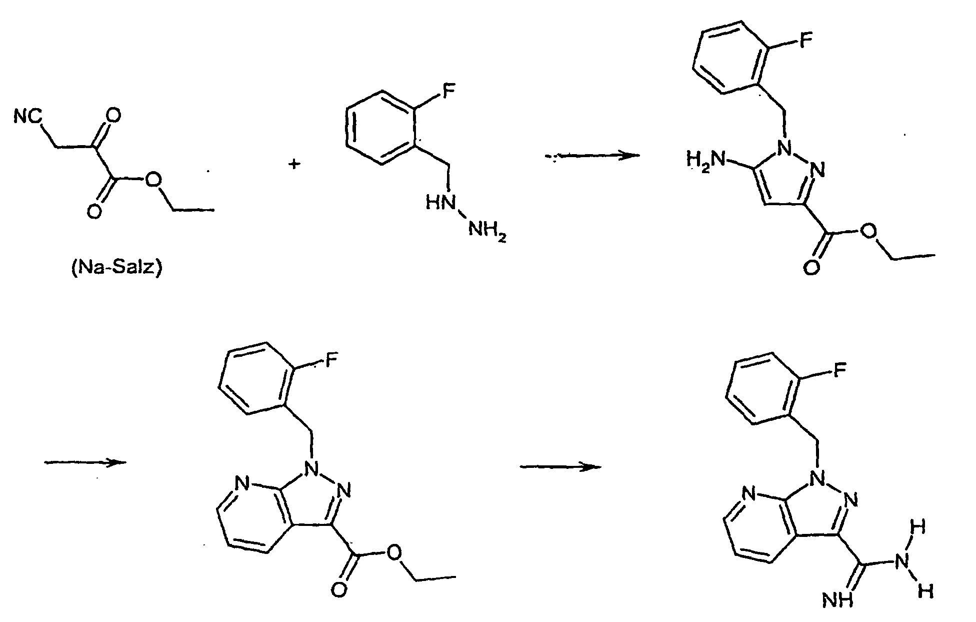

Die Verbindung der Formel (II) ist in einer mehrstufigen Synthese aus dem literaturbekannten Natriumsalz des Cyanobrenztraubensäureethylesters (Borsche und Manteuffel, Liebigs. Ann. Chem. 1934, 512, 97) erhältlich. Durch dessen Umsetzung mit 2-Fluorbenzylhydrazin unter Erhitzen und Schutzgasatmosphäre in einem inerten Lösungsmittel wie Dioxan erhält man den 5-Amino-1-(2-fluorbenzyl)-pyrazol-3-carbonsäureethylester, der durch Umsetzung mit Dimethylaminoacrolein oder 1,1,3,3-Tetramethoxypropan im sauren Medium unter Schutzgasatmosphäre und Erhitzen zum entsprechenden Pyridinderivat cyclisiert. Dieses Pyridinderivat 1-(2-Fluorbenzyl)-1H-pyrazolo[3,4-b]pyridin-3-carbonsäureethylester wird durch eine mehrstufige Sequenz, bestehend aus Überführung des Esters mit Ammoniak in das entsprechende Amid, Dehydratisierung mit einem wasserentziehenden Mittel wie Trifluoressigsäureanhydrid zum entsprechenden Nitrilderivat, Umsetzung des Nitrilderivats mit Natriumethylat und abschließende Reaktion mit Ammoniumchlorid in die Verbindung der Formel (II) überführt.

Die Verbindung der Formel (III) kann aus den (z.B. bei Aldrich) käuflich erhältlichen Verbindungen t-Butoxybis(dimethylamino)methan und Methoxyacetonitril durch Umsetzung dieser Reaktanden vorzugsweise in äquimolaren Mengen gegebenenfalls in einem organischen inerten Lösungsmittel wie einem cyclischen Ether, vorzugsweise Dioxan, vorzugsweise bei Normaldruck und Rühren der Reaktionslösung für mehrere Stunden, beispielsweise 12 Stunden, ber erhöhter Temperatur, beispielsweise 60-110°C, vorzugsweise 70-90°C, insbesondere 80°C hergestellt werden.

Die Umsetzung der Verbindungen der Formeln (II) und (III) zur Verbindung der Formel (IV) kann durch Einsatz der Reaktanden in äquimolaren Mengen beziehungsweise unter Verwendung der Verbindung der Formel (III) im leichten Überschuss in einem organischen Lösungsmittel, beispielsweise einem Alkohol, vorzugsweise Isoamylalkohol in Gegenwart einer geringen Menge einer Base, beispielsweise einem organischen Amin, insbesondere Piperidin, vorzugsweise bei Normaldruck und Rühren der Reaktionslösung für mehrere Stunden, beispielsweise 12 Stunden, bei erhöhter Temperatur, beispielsweise 60-130°C, vorzugsweise 80-120°C, insbesondere 110°C, und anschließende Freisetzung der Hydroxygruppe durch Umsetzung der so erhaltenen Verbindung mit einer vorzugsweise äquimolaren Menge eines Thiols wie beispielsweise Thiophenol in Gegenwart einer geringen Menge einer Base wie einer Alkalimetallbase, beispielsweise einem Alkalimetallcarbonat, vorzugsweise Kaliumcarbonat in einem organischen Lösungsmittel wie beispielsweise 1-Methyl-2-pyrrolidon vorzugsweise bei Normaldruck und Rühren der Reaktionslösung für mehrere Stunden, beispielsweise 1 Stunde, bei erhöhter Temperatur, beispielsweise 100-200°C, vorzugsweise 150-200°C, durchgeführt werden.

Die Verbindungen der Formel (IV) können mit Verbindungen der Formel X-CONR³R⁴, wobei X, R³ und R⁴ wie vorstehend definiert sind, zu den erfindungsgemäßen Verbindungen der Formel (I) umgesetzt werden. Diese Carbamoylderivate sind entweder käuflich erhältlich oder auf dem Fachmann bekannte Weise zugänglich. Beispielhaft sei hier die Umsetzung entsprechender sekundärer Amine mit Phosgen oder Phosgenersatzmitteln wie Chlorameisensäuretrichlormethylester (Diphosgen) oder Bis(trichlormethyl)carbonat (Triphosgen) genannt (vgl. J. March, Advanced Organic Synthesis, 3^{rd} ed., Wiley 1985, 370, D. Hoppe, Synthesis 1996, 149-154). Die hierfür benötigten sekundären Amine sind entweder käuflich erhältlich oder auf dem Fachmann bekannte Weise zugänglich, beispielsweise durch Umsetzung eines primären-Amins mit einem entsprechenden Aldehyd oder Keton unter Verwendung eines für derartige Reaktionen herkömmlich eingesetzten Reduktionsmittels, beispielsweise einem Metallhydridkomplex, vorzugsweise einem Alkalimetallhydridkomplex wie Natriumcyanoborhydrid ("Reduktive Aminierung", vgl. K.-L. Yu, J. Ostrowski, P. Reczek, M.M. Mansuri, J.E. Starrett Jr. , Synthetic Communications, 1995, **25**, 2819-2827).

Die Umsetzung der Verbindungen der Formel (IV) mit Verbindungen der Formel X-CONR³R⁴, wobei X, R³ und R⁴ wie vorstehend definiert sind, zu den erfindungsgemäßen Verbindungen der Formel (I) erfolgt vorzugsweise entweder in einem für derartige Reaktionen herkömmlich verwendeten organischen Lösungsmittel wie beispielsweise einem cyclischen Ether, insbesondere Tetrahydrofuran (THF), in Gegenwart einer äquimolaren Menge oder eines leichten Überschusses einer Base, vorzugsweise einer Alkalimetallbase, bevorzugt eines Alkalimetallhydrids und insbesondere bevorzugt Natriumhydrid vorzugsweise bei Normaldruck und Rühren der Reaktionslösung für mehrere Stunden, beispielsweise 12 Stunden, bei Raumtemperatur. Die Reaktanden werden hierbei äquimolar oder das Carbamoylderivat im leichten Überschuss eingesetzt. Eine andere Möglichkeit besteht in der Durchführung der Reaktion in Pyridin ohne weitere Base vorzugsweise bei Normaldruck und Rühren der Reaktionslösung für mehrere Stunden, beispielsweise 2 Stunden, bei erhöhter Temperatur von 60 bis 130°C, vorzugsweise 80 bis 120°C und insbesondere bei 110°C.

Analog kann auch die Umsetzung der Verbindung der Formel (IV) mit Thiocarbamoylchloriden zu den entsprechenden Carbamaten erfolgen. Bevorzugt ist die Umsetzung in Pyridin. Die erfindungsgemäß verwendeten Thiocarbamoylchloride sind käuflich erhältlich.

Anschließend können gegebenenfalls am Molekül vorhandene Schutzgruppen auf dem Fachmann bekannte Weise entfernt werden. Dies bezüglich sei beispielsweise auf T.W. Greene, P.G.M. Wuts, Protective Groups in Organic Synthesis, second edition, New York, 1991, bezüglich möglicher Schutzgruppen und deren Entfernung aus der Zielverbindung verwiesen.

Die erfindungsgemäßen Verbindungen der allgemeinen Formel (I) zeigen ein nicht vorhersehbares, wertvolles pharmakologisches Wirkspektrum.

Die erfindungsgemäßen Verbindungen der allgemeinen Formel (I) führen zu einer Gefäßrelavation, Thrombozytenaggregationshemmung und zu einer Blutdrucksenkung sowie zu einer Steigerung des koronaren Blutflusses. Diese Wirkungen sind über eine direkte Stimulation der löslichen Guanylatzyklase und einem intrazellulären cGMP-Anstieg vermittelt. Außerdem verstärken die erfindungsgemäßen Verbindungen der allgemeinen Formel (I) die Wirkung von Substanzen, die den cGMP-Spiegel steigern, wie beispielsweise EDRF (Endothelium derived relaxing factor), NO-Donatoren, Protoporphyrin IX, Arachidonsäure oder Phenylhydrazinderivate.

Sie können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, peripheren und kardiälen Gefäßerkrankungen, von Arrhythmien, zur Behandlung von thromboembolischen Erkrankungen und Ischämien wie Myokardinfarkt, Himschlag, transistorisch und ischämische Attacken, periphere Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose, asthmatischen Erkrankungen und Krankheiten des Urogenitalsystems wie beispielsweise Prostatahypertrophie, erektile Dysfunktion, weibliche sexuelle Dysfunktion, Osteoporose, Gastroparese und Inkontinenz eingesetzt werden.

Die in der vorliegenden Erfindung beschriebenen Verbindungen der allgemeinen Formel (I) stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung, oder Gedächtnisleistung nach kognitiven Störungen, wie sie insbesondere bei Situationen/Krankheiten/Syndromen auftreten wie "Mild cognitive impairment", Altersassozüerte Lern- und Gedächtnisstörungen, Altersassoziierte Gedächtnisverluste, Vaskuläre Demenz, Schädel-Hirn-Trauma, Schlaganfall, Demenz, die nach Schlaganfällen auftritt ("post stroke dementia"), post-traumatisches Schädel Hirn Trauma, allgemeine Kanzentrationsstörungen, Konzentrationsstörungen in Kindern mit Lem-und Gedächtnisproblemen, Alzheimersche Krankheit, Vaskuläre Demenz, Demenz mit Lewy-Körperchen, Demenz mit Degeneration der Frontallappen einschließlich des Pick's Syndroms, Parkinsonsche Krankheit, Progressive nuclear palsy, Demenz mit corticobasaler Degeneration, Amyolateralsklerose (ALS), Huntingtonsche Krankheit, Multiple Sklerose, Thalamische Degeneration, Creutzfeld-Jacob-Demenz, HIV-Demenz, Schizophrenie mit Demenz oder Korsakoff-Psychose. Sie eignen sich auch zur Behandlung von Erkrankungen des Zentralnervensystems wie Angst-, Spannungsund Depressionszuständen, zentralnervös bedingten Sexualdysfunktionen und Schlafstörungen, sowie zur Regulierung krankhafter Störungen der Nahnmgs-, Genuss- und Suchtmittelaufnahme.

Weiterhin eignen sich die Wirkstoffe auch zur Regulation der cerebralen Durchblutung und stellen somit wirkungsvolle Mittel zur Bekämpfung von Migräne dar.

Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen der allgemeinen Formel (I) zur Bekämpfung von Schmerzzuständen eingesetzt werden.

Zudem besitzen die erfindungsgemäßen Verbindungen antiinflammatorische Wirkung und können daher als entzündungshemmende Mittel eingesetzt werden.

Darüber hinaus umfasst die Erfindung die Kombination der erfindungsgemäßen Verbindungen der allgemeinen Formel (I) mit organischen Nitraten und NO-Donatoren.

Organische Nitrate und NO-Donatoren im Rahmen der Erfindung sind im allgemeinen Substanzen, die über die Freisetzung von NO bzw. NO-Species ihre therapeutische Wirkung entfalten. Bevorzugt sind Natriumnitroprussid, Nitroglycerin, Isosorbiddinitrat, Isosorbidmononitrat, Molsidomin und SIN-1.

Außerdem umfasst die Erfindung die Kombination mit Verbindungen, die den Abbau von cyclischem Guanosinmonophosphat (cGMP) inhibieren. Dies sind insbesondere Inhibitoren der Phosphodiesterasen 1, 2 und 5; Nomenklatur nach Beavo und Reifsnyder (1990) TiPS 11 S. 150 bis 155. Durch diese Inhibitoren wird die Wirkung der erfindungsgemäßen Verbindung potenziert und der gewünschte pharmakologische Effekt gesteigert.

### Biologische Untersuchungen

### Gefäßrelaxierende Wirkung in vitro

Kaninchen werden durch Nackenschlag betäubt und entblutet. Die Aorta wird entnommen, von anhaftendern Gewebe befreit, in 1,5 mm breite Ringe geteilt und einzeln unter einer Vorspannung in 5 ml-Organbäder mit 37°C warmer, carbogenbegaster Krebs-Henseleit-Lösung folgender Zusammensetzung (mM) gebracht: NaCl: 119; KCl: 4,8; CaCl₂ x 2 H₂O: 1; MgSO₄ x7 H₂O; 1,4; KH₂PO₄: 1,2; NaHCO₃:25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung untersucht und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀). Das Standardapplikationsvolumen beträgt 5 µl, der DMSO-Anteil in der Badlösung entspricht 0,1 %. Die Ergebnisse sind nachstehend in Tabelle 1 aufgeführt:

**Tabelle 1:**

| **Gefäßrelaxierende Wirkung in vitro** | |
|---|---|
| Beispiel Nr. | IC₅₀ [µM] |
| 2 | 0,65 |
| 6 | 0,27 |
| 11 | 0,52 |
| 15 | 0,32 |
| 19 | 0,42 |
| 26 | 0,34 |

### Bestimmung der Leberclearance in vitro

Ratten werden anästhesiert, heparinisiert, und die Leber in situ über die Pfortader perfundiert. Ex vivo werden dann aus der Leber mittels Collagenase-Lösung die primären Ratten-Hepatozyten gewonnen. Es wurden 2^{·}10⁶ Hepatozyten pro ml mit jeweils der gleichen Konzentration der zu untersuchenden Verbindung bei 37°C inkubiert. Die Abnahme des zu untersuchenden Substrates über die Zeit wurde bioanalytisch (HPLC/UV, HPLC/Fluoreszenz oder LC/MSMS) an jeweils 5 Zeitpunkten im Zeitraum von 0-15 min nach Inkubationsstart bestimmt. Daraus wurde über Zellzahl und Lebergewicht die Clearance errechnet.

### Bestimmung der Plasmaclearance in vivo

Die zu untersuchende Substanz wird Ratten über die Schwanzvene intravenös als Lösung appliziert. Zu festgelegten Zeitpunkten wird den Ratten Blut entnommen, dieses wird heparinisiert und durch herkömmliche Maßnahmen Plasma daraus gewonnen. Die Substanz wird im Plasma bioanalytisch quantifiziert. Aus den so ermittelten Plasmakonzentrations-Zeit-Verläufen werden über herkömmliche hierfür verwendete nicht-kompartimentelle Methoden die pharmakokinetischen Parameter errechnet.

Zur vorliegenden Erfindung gehören pharmazeutische Zubereitungen, die neben nichttoxischen, inerten pharmazeutisch geeigneten Trägerstoffen die erfindungsgemäßen Verbindungen der allgemeinen Formel (I) enthält sowie Verfahren zur Herstellung dieser Zubereitungen.

Die Wirkstoff können gegebenenfalls in einem oder mehreren der oben angegebenen Trägerstoffe auch in mikroverkapselter Form vorliegen.

Die therapeutisch wirksamen Verbindungen der allgemeinen Formel (I) sollen in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen der allgemeinen Formel (I) auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,01 bis etwa 700, vorzugsweise 0,01 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in. Mengen von etwa 0,1 bis etwa 80, insbesondere 0,1 bis 30 mg/kg Körpergewicht.

Die vorliegende Erfindung wird nachstehend anhand von nicht einschränkenden bevorzugten Beispielen näher dargestellt. Soweit nicht anderweitig angegeben, beziehen sich alle Mengenangaben auf Gewichtsprozente.

### Beispiele

### Abkürzungen:

- RT:: Raumtemperatur
- EE:: Essigsäureethylester
- MCPBA:: m-Chlorperoxybenzoesäure
- BABA:: n-Butylacetat/n-Butanol/Eisessig/Phosphatpuffer pH 6
(50:9:25.15; org. Phase)
- DMF:: N,N-Dimethylformamid

### Laufmittel für die Dünnschichtchromatographie:

- T1 E1:: Toluol - Essigsäureethylester (1:1)
- T1 EtOH1 :: Toluol - Methanol (1:1)
- C1 E1:: Cyclohexan - Essigsäureethylester (1:1)
- C1 E2:: Cyclohexan - Essigsäureethylester (1:2)

### Methoden zur Ermittlung der HPLC-Retentionszeiten:

### Methode A (HPLC-MS):

Eluent: A= CH₃CN B=0.6 g 30%ige HCl/l H₂O
Fluss: 0.6 ml/min
Säulenofen: 50°C
Säule: Symmetry C18 2.1*150mm
Gradient:

| Zeit (min) | %A | %B | Fluss (ml/min) |
|---|---|---|---|
| 0 | 10 | 90 | 0.6 |
| 4 | 90 | 10 | 0.6 |
| 9 | 90 | 10 | 0.8 |

### Methode B (HPLC):

Eluent: A=5 ml HClO₄/l H₂O, B=CH₃CN
Fluss: 0.75 ml/min
L-R Temperatur: 30.00°C 29.99°C
Säule: Kromasil C18 60*2mm
Gradient:

| Zeit (min) | %A | %B |
|---|---|---|
| 0.50 | 98 | 2 |
| 4.50 | 10 | 90 |
| 6.50 | 10 | 90 |
| 6.70 | 98 | 2 |
| 7.50 | 98 | 2 |

### Methode C (HPLC):

Eluent: A= H₃PO₄ 0.01 mol/l, B=CH₃CN
Fluss: 0.75 ml/min
L-R Temperatur: 30.01°C 29.98°C
Säule: Kromasil C18 60*2mm
Gradient:

| Zeit (min) | %A | %B |
|---|---|---|
| 0.00 | 90 | 10 |
| 0.50 | 90 | 10 |
| 4.50 | 10 | 90 |
| 8.00 | 10 | 90 |
| 8.50 | 90 | 10 |
| 10.00 | 90 | 10 |

### Methode D (chirale HPLC):

- Eluent:: 50% iso-Hexan, 50% Ethanol
- Fluss:: 1.00 ml/min:
- Temperatur:: 40°C
- Säule:: 250*4,6 mm, gefüllt mit Chiralcel OD, 10 µm

### Methode E (HPLC-MS):

Eluent: A= CH₃CN B=0.3 g 30%ige HCl/1 H₂O
Fluss: 0.9 ml/min
Säulenofen: 50°C
Säule: Symmetry C18 2.1*150mm
Gradient:

| Zeit (min) | %A | %B | Fluss (ml/min) |
|---|---|---|---|
| 0 | 10 | 90 | 0.9 |
| 3 | 90 | 10 | 1.2 |
| 6 | 90 | 10 | 1.2 |

### Methode F:

Eluent: A= CH₃CN + 0,1% HCOOH B= H₂O + 0,1% HCOOH
Säulenofen: 40°C
Säule: Symmetry C18 2.1*150mm
Gradient:

| Zeit (min) | %A | %B | Fluss (ml/min) |
|---|---|---|---|
| 0 | 10 | 90 | 0.5 |
| 4 | 90 | 10 | 0,5 |
| 6 | 90 | 10 | 0,5 |
| 6,1 | 10 | 90 | 1,0 |
| 7,5 | 10 | 90 | 0,5 |

### Ausgangsverbindungen:

### I. Synthese von 3,3-Bis(dimethylamino)-2-methoxypropionitril

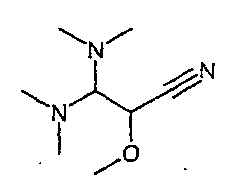

40.0 g (229.5 mmol) ter-Butoxybis(dimethylamino)methan und 16.3 g (229.5 mmol) Methoxyacetonitril werden über Nacht bei 80°C gerührt. Zur Aufarbeitung wird flüchtiges Material am Rotationsverdampfer abgezogen und der Rückstand im Kugelrohr bei 140°C im Hochvakuum destilliert. Das Produkt enthält laut NMR-Spektrum (300 MHz, D₆-DMSO) das Enamin als E/Z-Gemisch, das durch Eliminierung von Dimethylamin entsteht. Die Produktmischung wird ohne weitere Reinigung in die nächste Reaktion eingesetzt.
- Ausbeute:: 24.7 g (60%)

### II. Synthese von 1-(2-Fluorbenzyl)1H-pyrazolo(3,4-b]pyridin-3-carboxamidin

### 2A) 5-Amino-1-(2-fluorbenzyl)-pyrazol-3-carbonsäureethylester

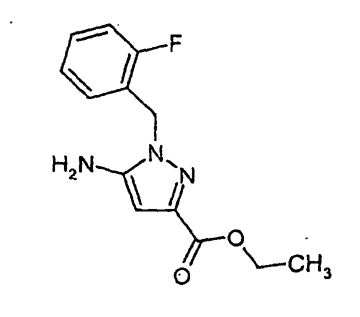

100 g (0.613 mol) Natriumsalz des Cyanobrenztraubensäureethylester (Darstellung analog Borsche und Manteuffel, Liebigs Ann. 1934, 512, 97) werden unter gutem Rühren unter Argon in 2.5 l Dioxan bei Raumtemperatur mit 111.75 g (75 ml, 0.98 mol) Trifluoressigsäure versetzt und 10 min gerührt, wobei ein großer Teil des Eduktes in Lösung geht. Dann gibt man 85.93 g (0.613 mol) 2-Fluorbenzylhydrazin hinzu und kocht über Nacht. Nach Abkühlen werden die ausgefallenen Kristalle des Natriumtrifluoracetats abgesaugt, mit Dioxan gewaschen und die Lösung roh weiter umgesetzt.

### 2B) 1-(2-Fluorbenzyl)-1H-pyrazolo[3,4-b]pyridin-3-carbonsäureethylester

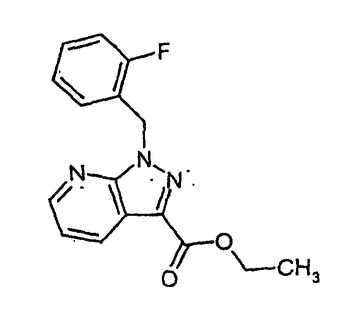

Die aus 2A) erhaltene Lösung wird mit 61.25 ml (60.77 g, 0.613 mol) Dimethylaminoacrolein und 56.28 ml (83.88 g, 0.736 mol) Trifluoressigsäure versetzt und unter Argon 3 Tage lang gekocht. Anschließend wird das Lösungsmittel im Vakuum verdampft, der Rückstand in 2 l Wasser gegeben und dreimal mit je 1 l Essigester extrahiert. Die vereinigten organischen Phasen werden mit Magnesiumsulfat getrocknet und einrotiert. Man chromatographiert auf 2.5 kg Kieselgel und eluiert mit einem Toluol / Toluol-Essigester=4:1 -Gradienten. Ausbeute: 91.6 g (49.9 % d.Th. über zwei Stufen).
Smp. 85 °C
R_{f} (SiO₂, T1E1): 0.83

### 2 C) 1-(2-Fluorbenzyl)-1H-pyrazolo[3,4-b]pyridin-3-carboxamid

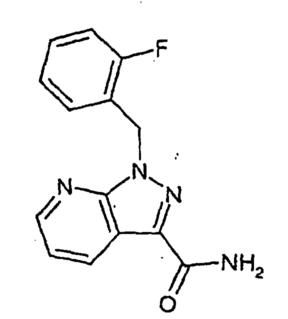

10.18 g (34 mmol) des in Beispiel 2B) erhaltenen Esters werden in 150 ml mit Ammoniak bei 0 - 10°C gesättigtem Methanol vorgelegt. Man rührt zwei Tage bei Raumtemperatur und engt anschließend im Vakuum ein.
R_{f} (SiO₂, T1E1): 0.33.

### 2D) 3-Cyano-1-(2-fluorbenzyl)-1H-pyrazolo[3,4-b]pyridin

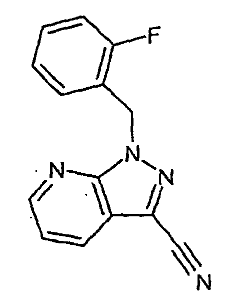

36.1 g (133 mmol) 1-(2-Fluorbenzyl)-1H-pyrazolo[3,4-b]pyridin-3-carboxamid aus Beispiel 2C) werden in 330 ml THF gelöst und mit 27 g (341 mmol) Pyridin versetzt. Anschließend gibt man innerhalb von 10 min 47.76 ml (71.66 g, 341 mmol) Trifluoressigsäureanhydrid hinzu, wobei die Temperatur bis auf 40 °C ansteigt. Man rührt über Nacht bei Raumtemperatur. Anschließend wird der Ansatz in 11 Wasser gegeben und dreimal mit je 0.5 1 Essigester extrahiert. Die organische Phase wird mit gesättigter Natriumhydrogencarbonatlösung und mit 1 N HCl gewaschen, mit MgSO₄ getrocknet und einrotiert.
Ausbeute: 33.7 g (100% d.Th.)
Smp: 81°C
R_{f} (SiO₂, T1E1): 0.74

### 2E) (2-Fluorbenzyl)-1H-pyrazolo[3,4-b]pyridin-3-carboximidsäuremethylester

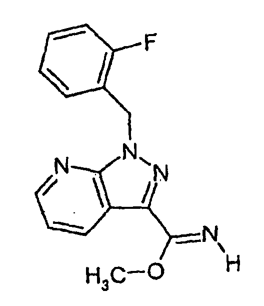

Man löst 30.37 g (562 mmol) Natriummethylat in 1.5 l Methanol und gibt 36.45 g (144.5 mmol) 3-Cyano-1-(2-fluorbenzyl)-1H-pyrazolo[3,4-b]pyridin (aus Beispiel 2D) hinzu. Man rührt 2 Stunden bei Raumtemperatur und setzt die erhaltene Lösung direkt für die nächste Stufe ein.

### 2F) 1-(2-Fluorbenzyl)1H-pyrazolo[3, 4-b]pyridin-3-carboxamidin

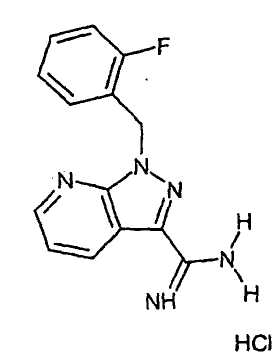

Die aus Beispiel 2E) erhaltene Lösung von (2-Fluorbenzyl)-1H-pyrazolo[3,4-b]pyridin-3-carboximidsäuremethylester in Methanol wird mit 33.76 g (32.19 ml, 562 mmol) Eisessig und 9.28 g (173 mmol) Ammoniumchlorid versetzt und über Nacht unter Rückfluss gerührt. Man verdampft das Lösungsmittel im Vakuum, verreibt den Rückstand gut mit Aceton und saugt den ausgefallenen Feststoff ab.
¹H-NMR (d₆-DMSO, 200 MHz): δ= 5,93 (s, 2H); 7,1-7,5 (m, 4 H); 7,55 (dd, 1H); 8,12 (dd, 1H); 8,30 (dd, 1H); 9,5 (bs, 4H-austauschbar) ppm.
MS (EI): m/z = 270,2 (M-HCl).

### III. Synthese von 2-[1-(2-Fluorobenzyl)-1H-pyrazolo[3,4-b]pyridin-3-yl]-5-methoxy-4-pyrimidinylamin

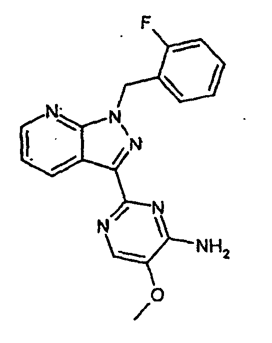

46.8 g (134.8 mmol) 1-(2-Fluorobenzyl)-1H-pyrazolo[3,4-b]pyridin-3-carboximidamid aus Beispiel II werden in Isoamylalkohol gelöst. Dazu gibt man 24.7 g (144.2 mmol) 3,3-bis(dimethylamino)-2-methoxypropionitril aus Beispiel I und 1.15 g (1.33 ml, 13.5 mmol) Piperidin und lässt 3 Tage bei 110°C rühren. Zur Aufarbeitung kühlt man auf 0°C, saugt das ausgefallene Produkt ab, wäscht gut mit kaltem Diethylether und trocknet im Vakuumtrockenschrank bei 50°C.
- Ausbeute:: 25.4 g (52.7%)
- R_{f}-Wert:: 0.34 (Dichlormethan/Methanol 20:1)
- ¹H-NMR:: (400 MHz, D₆-DMSO), δ = 3.89 (2, 3H, OCH₃), 5.79 (s, 2H, CH₂), 6.93 (br. s, 2H, NH₂), 7.10-7.26 (m, 3H, Ar-H), 7.31-7.39 (m, 2H, Ar-H), 7.98 (s, 1H, Pyrimidin-H), 8.61 (dd, 1H, Pyridin-H), 8.92 (dd, 1H, Pyridin-H)
- MS:: (ESI pos.), *m*/*z* = 350.9 ([M+H]⁺), 700.8 ([2M+H]⁺)

### IV. Synthese von 4-Amino-2-[1-(2-fluorobenzyl)-1H-pyrazolo[3,4-b]pyridin-3-yl]-5-pyrimidinol

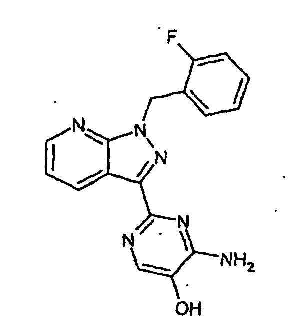

25.3 g (72.2 mmol) 2-[1-(2-fluorobenzyl)-1H-pyrazolo[3,4-b]pyridin-3-yl]-5-methoxy-4-pyrimidinylamin aus Beispiel III werden in 500 ml 1-Methyl-2-pyrrolidon gelöst. Dazu gibt man 7.96 g (7.42 ml, 72.2 mmol) Thiophenol und 2.50 g (18.1 mmol) Kaliumcarbonat und lässt ca. 1h bei 190°C rühren. Zur Aufarbeitung wird das Lösungsmittel abkondensiert, der Rückstand mit halbkonz. Ammoniumchlorid-Lösung versetzt und dreimal mit Ethylacetat extrahiert. Dabei fällt das Produkt größtenteils aus. Es wird abgesaugt und im Vakuumtrockenschrank bei 50°C getrocknet.
- Ausbeute:: 18. 1 g (72.3 %)
- R_{f}-Wert:: 0.44 (Dichlormethan/Methanol 10:1)
- ¹H-NMR:: (300 MHz, D₆-DMSO), δ = 5.78 (s, 2H, CH₂), 6.66 (br. s, 2H, NH₂), 7.09-7.38 (m, 5H, Ar-H), 7.82 (s, 1H, Pyrimidin-H), 8.60 (dd, 1H, Pyridin-H), 8.92.(dd, 1H, Pyridin-H), 9.4-10.2 (br. s, 1H, 0H)
- MS:: (ESI pos.), *m*/*z* = 337.3 ([M+H]⁺), 673.3 ([2M+H]⁺)

### V. Synthese von 3,4-Dimethoxybenzyl(methyl)carbamoylchlorid

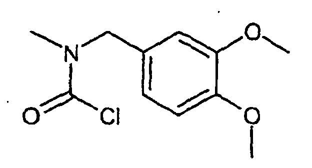

1.00 g (5.52 mmol) 3,4-Dimethoxybenzyl-N-methylamin (erhältlich durch reduktive Aminierung aus 3,4-Dimethoxybenzaldehyd) werden in 20 ml wasserfreiem Pyridin gelöst. Dazu gibt man 0.60 g (0.37 ml, 3.04 mmol) Chlorameisensäuretrichlormethylester ("Diphosgen") und lässt über Nacht bei Raumtemperatur rühren. Die Lösung wird ohne Aufarbeitung direkt in die nächste Stufe eingesetzt.

### VI. 4-Amino-2-[1-(2-fluorobenzyl)-1H-pyrazolo[3,4-b]pyridin-3-yl]-5-pyrimidinyl-3,4-dimethoxybenzyl(methyl)carbamat

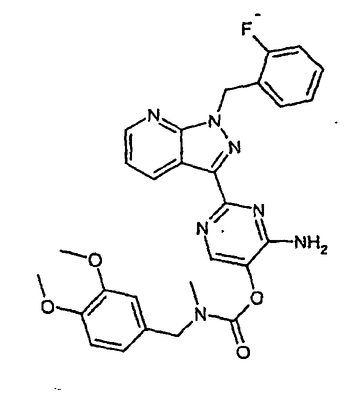

1.38 g ( 4.10 mmol) ) 4-Amino-2-[1-(2-fluorobenzyl)-1H-pyrazolo[3,4-b]pyridin-3-yl]-5-pyrimidinol aus Beispiel IV werden in 100 ml wasserfreiem Pyridin suspendiert und auf 110°C erhitzt. Dazu gibt man eine Lösung aus 1.20 g (4.92 mmol) 3,4-Dimethoxybenzyl(methyl)carbamoylchlorid aus Beispiel V in 20 ml wasserfreiem Pyridin (aus der zuvor beschriebenen Reaktion) und lässt bei ca. 5h bei 110°C rühren. Zur Aufarbeitung engt man die Reaktionslösung am Rotationsverdampfer ein und chromatographiert den Rückstand über Kieselgel mit Dichlormethan/Methanol (zunächst 100:1, dann 50:1). Das Produkt enthält laut NMR-Spektrum eine unbekannte Verunreinigung und wird ohne weitere Reinigung eingesetzt.
- Rf-Wert:: 0,90 (Dichlormethan/Methanol 10:1)

### VII. Synthese von Allyl(cyclopentyl)carbamoylchlorid

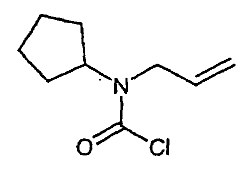

790 mg (0.48 ml, 3.99 mmol) Trichlormethylchloroformiat ("Diphosgen") werden in 15 ml Dichlormethan gelöst und auf 0°C gekühlt. Dazu tropft man langsam 1.00 g (1.17 ml, 7.986 mmol) Allyl(cyclopentyl)amin und 1.21 g (1.67 ml, 12.0 mmol) Triethylamin und lässt über Nacht bei Raumtemperatur rühren. Zur Aufarbeitung gießt man die Reaktionslösung auf Eiswasser, extrahiert dreimal mit Dichlormethan, trocknet über MgSO₄ und engt ein. Das Rohprodukt wird direkt in die nächste Sufe eingesetzt.
- MS:: (EI), *m*/*z* (%) = 187 ( 12, [M]⁺, Cl). 152 (30, [M-Cl]⁺), 120 (45), 69 (50), 41 *(100)*

### VIII. 4-Methoxybenzyl(2-methoxyethyl)carbamoylchlorid

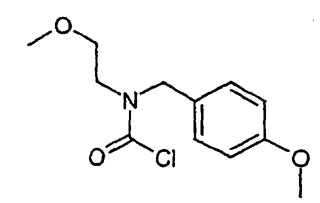

0.51 g (0.31 ml, 2.56 mmol) Chlorameisensäuretrichlonnethylester ("Diphosgen") werden in 15 ml Dichlormethan gelöst und langsam mit 1.00 g (5.12 mmol) 4-Methoxybenzyl(2-methoxyethyl)amin und 0.78 g (7.68 mmol) Triethylamin versetzt. Nach Rühren über Nacht bei Raumtemperatur gießt man auf Eiswasser, extrahiert dreimal mit Dichlormethan, trocknet die organische Phase über Magnesiumsulfat und engt am Rotationsverdampfer zur Trockne ein. Das Rohprodukt wird ohne Aufarbeitung direkt in die nächste Stufe eingesetzt.

### IX. 4-Amino-2-[1-(2-fluorobenzyl)-1H-pyrazolo [3,4-b] pyridin-3-yl]-5-pyrimidinyl 4-methoxybenzyl(2-methoxyethyl)carbamat

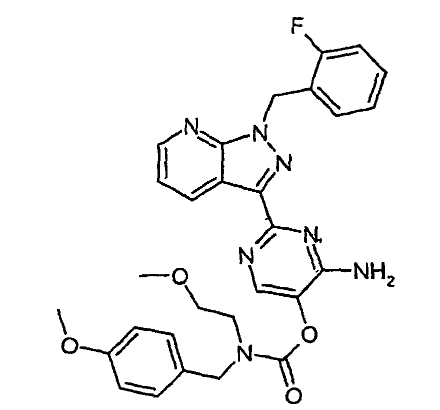

100 mg ( 0.30 mmol)) 4-Amino-2-[1-(2-fluorobenzyl)-1H-pyrazolo[3,4-b]pyridin-3-yl]-5-pyrimidinol aus Bsp. IV werden in 15 ml wasserfreiern Pyridin suspendiert und auf 110°C erhitzt. Dazu gibt man 92.0 mg (0.36 mmol) 4-Methoxybenzyl(2-methoxyethyl)carbamoylchlorid aus Bsp. VIII und lässt bei ca. 2h bei 110°C rühren. Zur Aufarbeitung engt man die Reaktionslösung am Rotationsverdampfer ein, nimmt den Rückstand in Dichlormethan auf, wäscht mit gesättigter Ammoniumchlorid-Lösung und mit gesättigter Natriumchlorid-Lösung, trennt die organische Phase ab und engt sie am Rotationsverdampfer ein. Chromatographie des Rückstands über Kieselgel mit Dichlozraethan/Methanol (100:1) lieferte das noch Produkt in 57%iger Reinheit und wurde ohne weitere Reinigung in die nächste Reaktion eingesetzt.
- Rf-Wert:: 0,86 (Dichlormethan/Methanol 10:1)

### Beispiele

Die Darstellung der Carbamate erfolgte mit Natriumhydrid in Tetrahydrofuran (THF) oder ohne Hilfsbase in Pyridin als Lösungsmittel. Für jeden der beiden Reaktionswege sind nachfolgend veranschaulichende Beispiele aufgeführt.

### 1. 4-Amino-2-[1-(2-fluorobenzyl)-1H-pyrazolo[3,4-b]pyridin-3-yl]-5-pyrimidinyl-1-pyrrolidincarboxylat

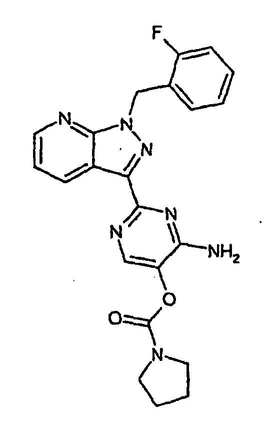

Zu einer Suspension von 50.0 mg (0.15.mmol) 4-Amino-2-[1-(2-fluorobenzyl)-1Hpyrazolo[3,4-b]pyridin-3-yl]-5-pyrimidinol aus Beispiel IV in 2 ml THF wurden bei RT 3.5 mg (0.15 mmol) Natriumhydrid zugegeben. Nachdem die Mischung 30 min bei RT gerührt wurde, wurden 21.8 mg (0.16 mmol) 1-Pyrrolidincarbonylchlorid zugegeben und die Mischung wurde über Nacht bei RT gerührt. Das Produkt wurde per Dünnschichtchromatographie gereinigt (Kieselgel, CH₂Cl₂/MeOH 10:1).
- Ausbeute.:: 50.4 g (78.2%)
- ¹H-NMR:: (400 MHz, DMSO-d₆): δ = 1.81 - 1.93 (m, 4H), 3.32 (t, J = 6.5 Hz, 2H),
3.57 t, J = 6.5 Hz, 2H), 5.81 (s, 2H), 7.05 - 7.30 (m, 5H), 7.31 - 7.44 (m, 2H), 8.12 (s, 1H), 8.63 (d, J = 4.4 Hz, 1H), 8.92 (d, J = 7.9 Hz, 1H).
- MS:: ESI pos.), *m*/*z* = 434.4 ([M+H]⁺)

Auf die gleiche Weise wurde erhalten:

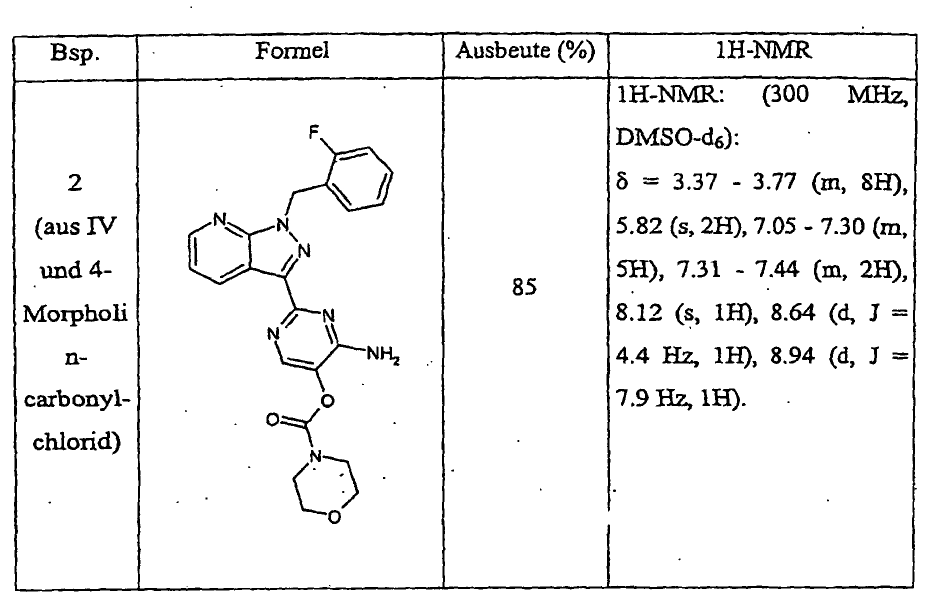

### 3. 4-Amino-2-[1-(2-ffuorobenzyl)-1H-pyrazolo[3,4-b]pyridin-3-yl]-5-pyrimidinyldiethylcarbamat

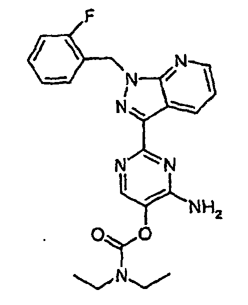

100 mg ( 0.297 mmol) ) 4-amino-2-[1-(2-fluorobenzyl)-1H-pyrazolo[3,4-b]pyridin-3-yl]-5-pyrimidinol aus Beispiel IV werden in 15 ml wasserfreiem Pyridin suspendiert und auf 110°C erhitzt. Dazu gibt man 48.4 mg (0.357 mmol) N,N-Diethylcarbamoylchlorid und rührt 2h bei 110°C. Anschließend engt man die Reaktionslösung am Rotationsverdampfer ein. Der Rückstand wird über Kieselgel mit Dichlormethan/Methanol 100:1 chromatographiert.
- Ausbeute:: 90.5 g (69.9 %)
- Rf-Wert:: 0.86 (Dichlormethan/Methanol 10:1)
- ¹H-NMR:: (200 MHz, D₆-DMSO), δ = 1.07-1.28 (m, 6H, *CH*₃CH₂), 3.22-3.53 (m, 4H, CH₃CH₂), 5.83 (s, 2H, CH₂), 7.10-7.42 (m, 7H, Ar-H und NH₂), 8.09 (s, 1H, Pyrimidin-H), 8.63 (dd, 1H, Pyridin-H), 8.94 (dd, 1H, Pyridin-H)
- MS:: (ESI pos.), *m*/*z* = 436.2 ([M+H]⁺), 871.0 ([2M+H]⁺)

Analog wurde hergestellt:

### 4. 1-{4-Amino-2-[1-(2-fluorobenzyl)-1H-pyrazolo[3,4-b]pyridin-3-yl]-5-pyrimidinyl} -4-tert-butyl-1,4-piperazindicarboxylat

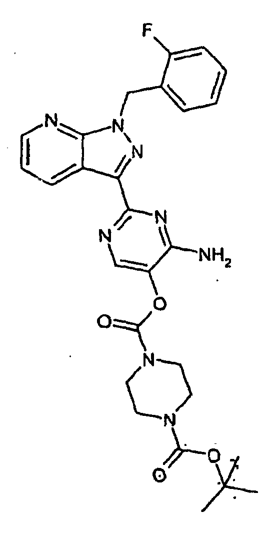

- Ausbeute:: 36.4 %
- Rf-Wert:: 0.77 (Dichlormethan/Methanol 10:1)
- ¹H-NMR:: (200 MHz, D₆-DMSO). Das Spektrum weißt E/Z-Rotamerie der Carbamat-Gruppe auf.
δ = 1.39 und 1.42 (2s, 9H, C(CH₃)₃), 3.09-3.14 (m, 1H, Piperazin-H), 3.26-3.34 (m, 1H, Piperazin-H), 3.36-3.48 (br.s, 6H, Piperazin-H), 3.52-3.64 (br.s, 2H, Piperazin-H), 5.81 (s, 2H, CH₂), 7.10-7.27 (m, 5H, Ar-H und NH₂), 7.30-7.40 (m, 2H, Ar-H), 8.12 (s, 1H, Pyrimidin-H), 8.63 (dd, 1H, Pyridin-H), 8.94 (dd, 1H, Pyridin-H)
- MS:: (ESI pos.), m/z = 549.1 ([M+H]⁺)

### Analog wurden hergestellt:

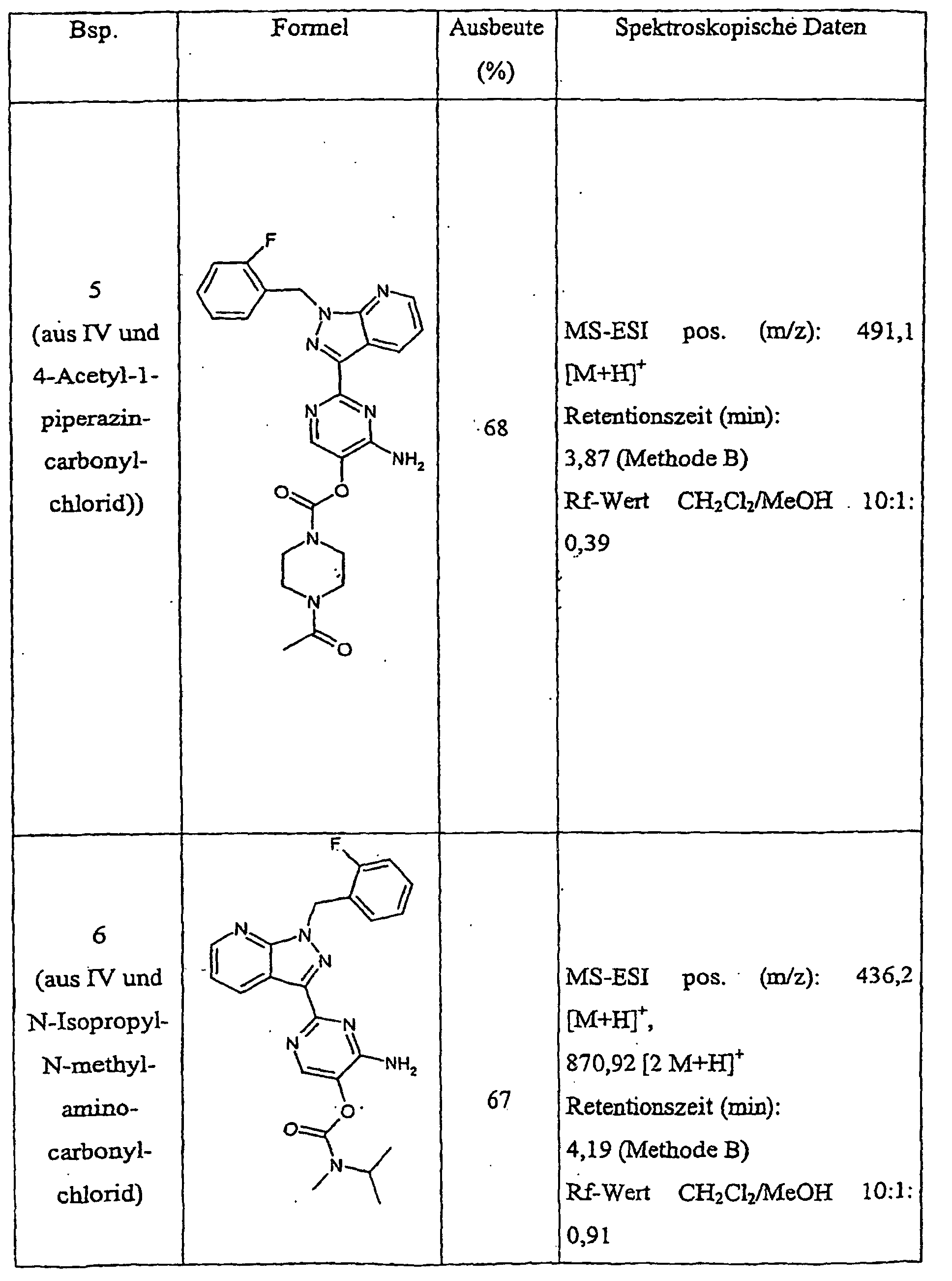

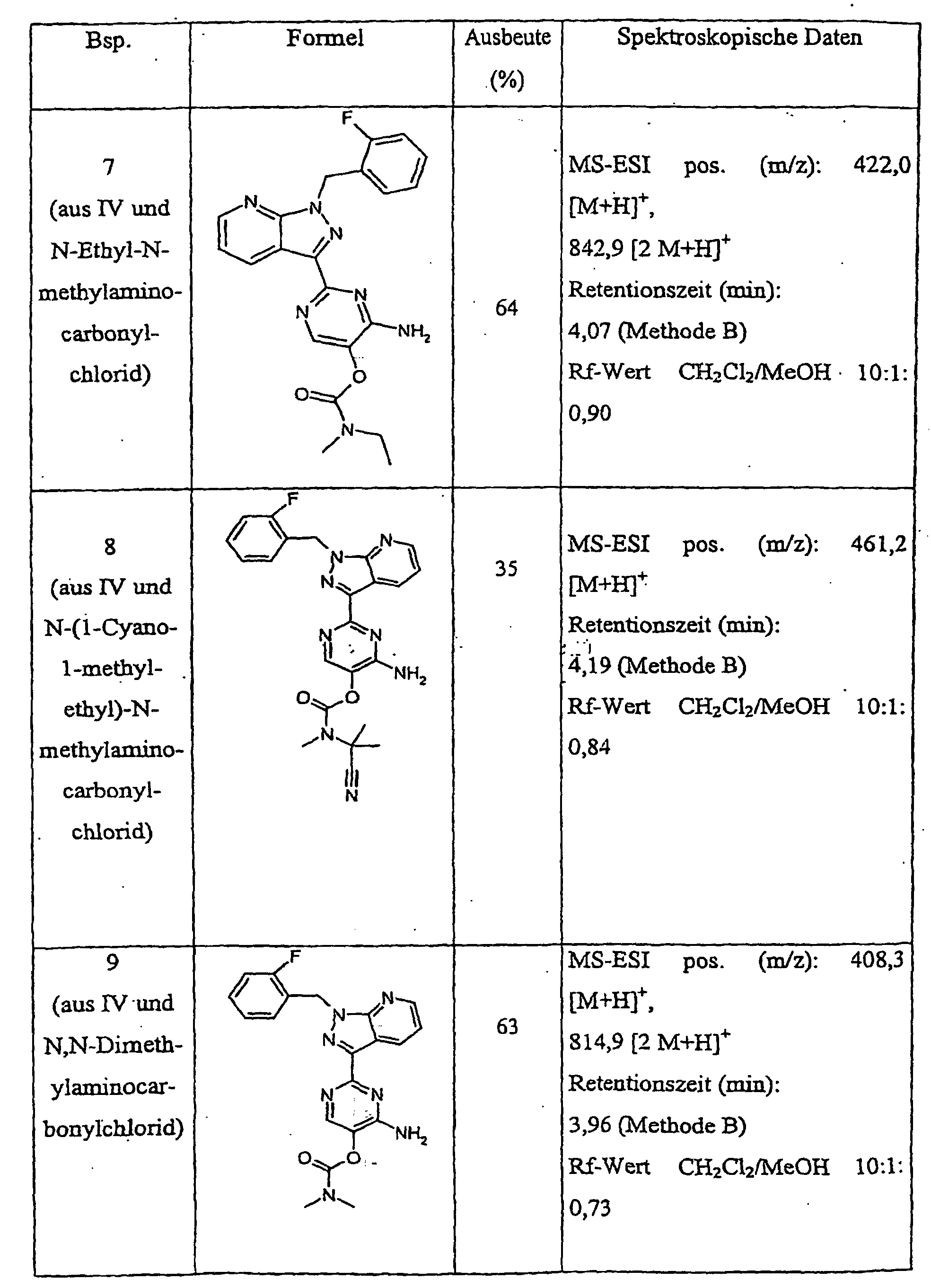

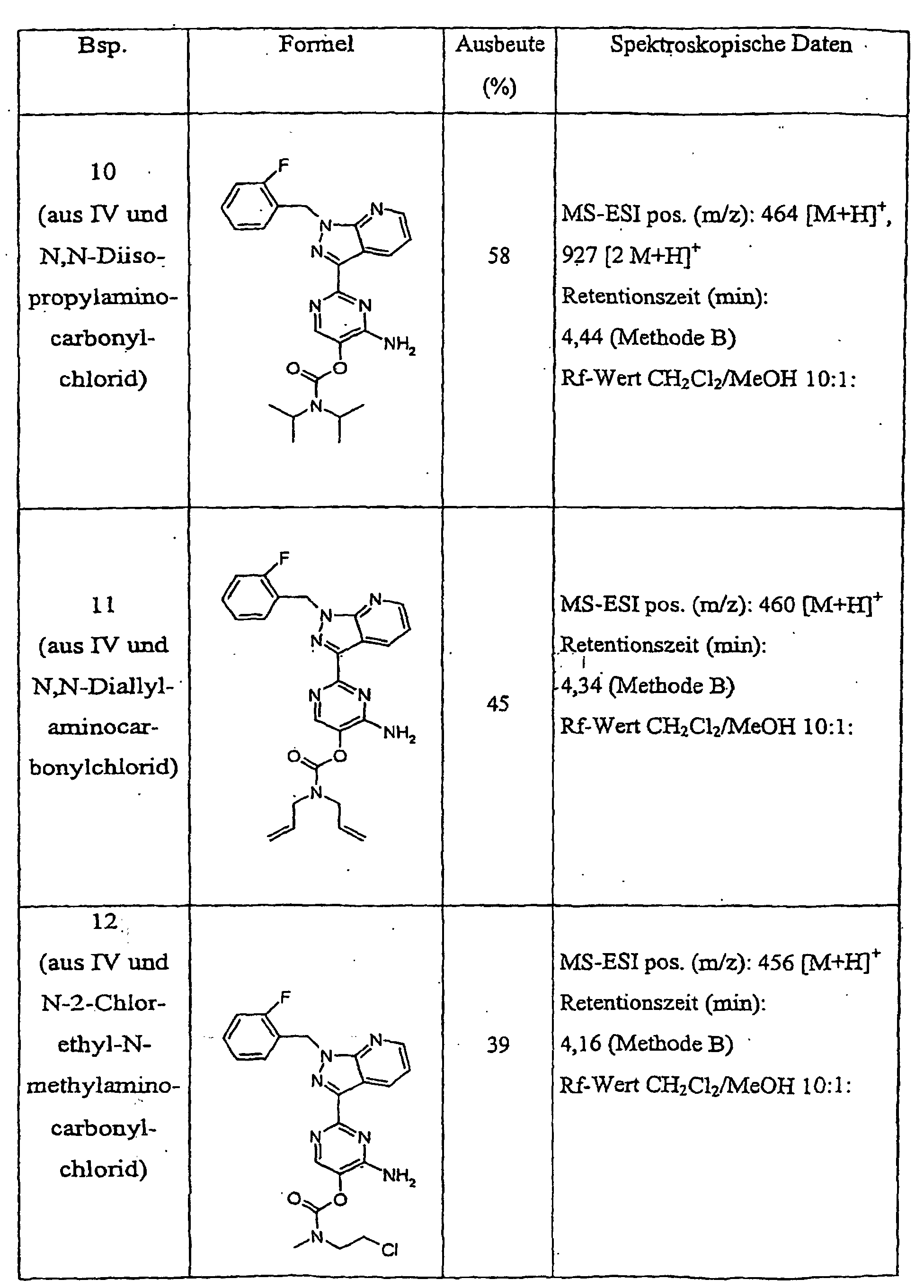

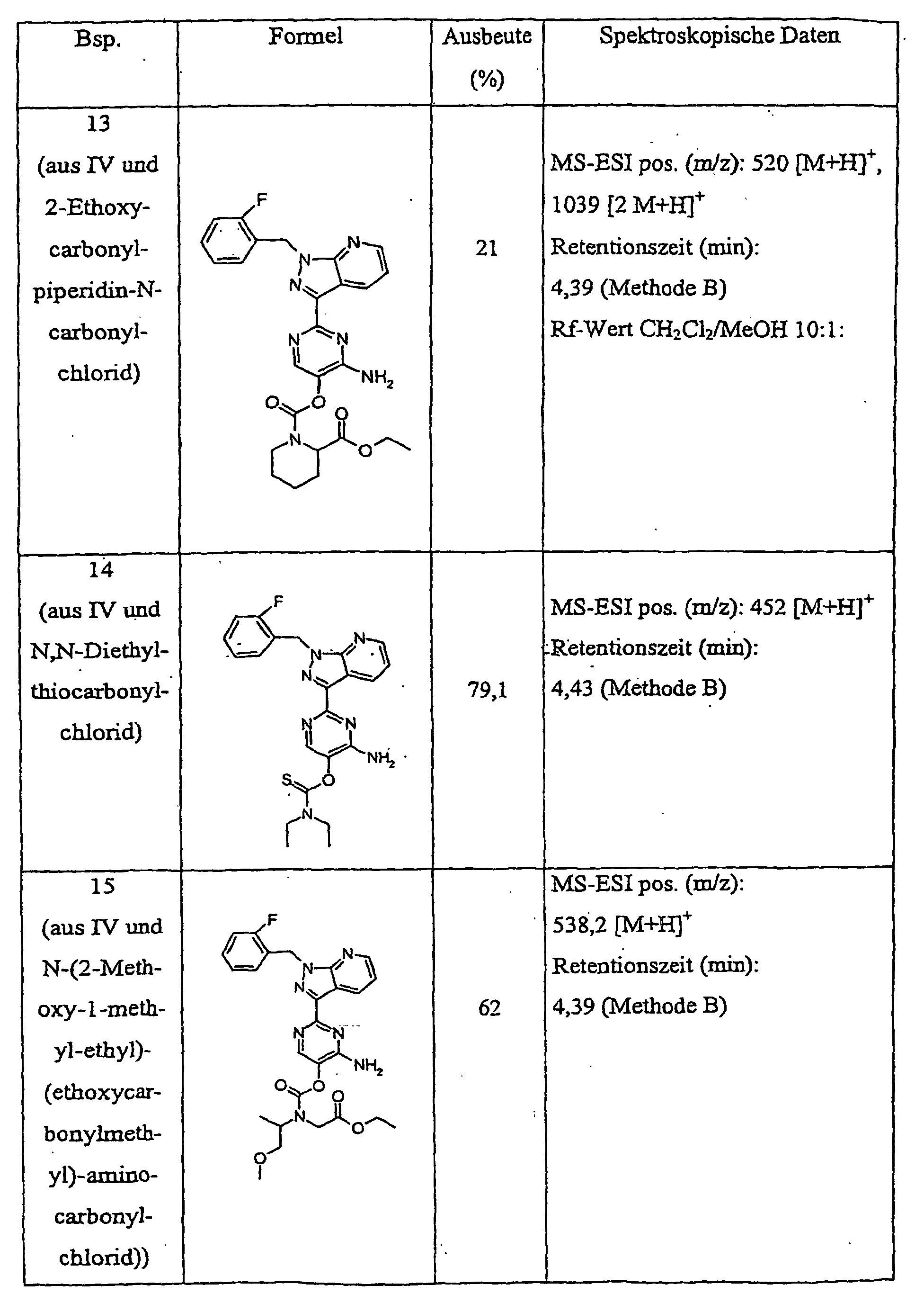

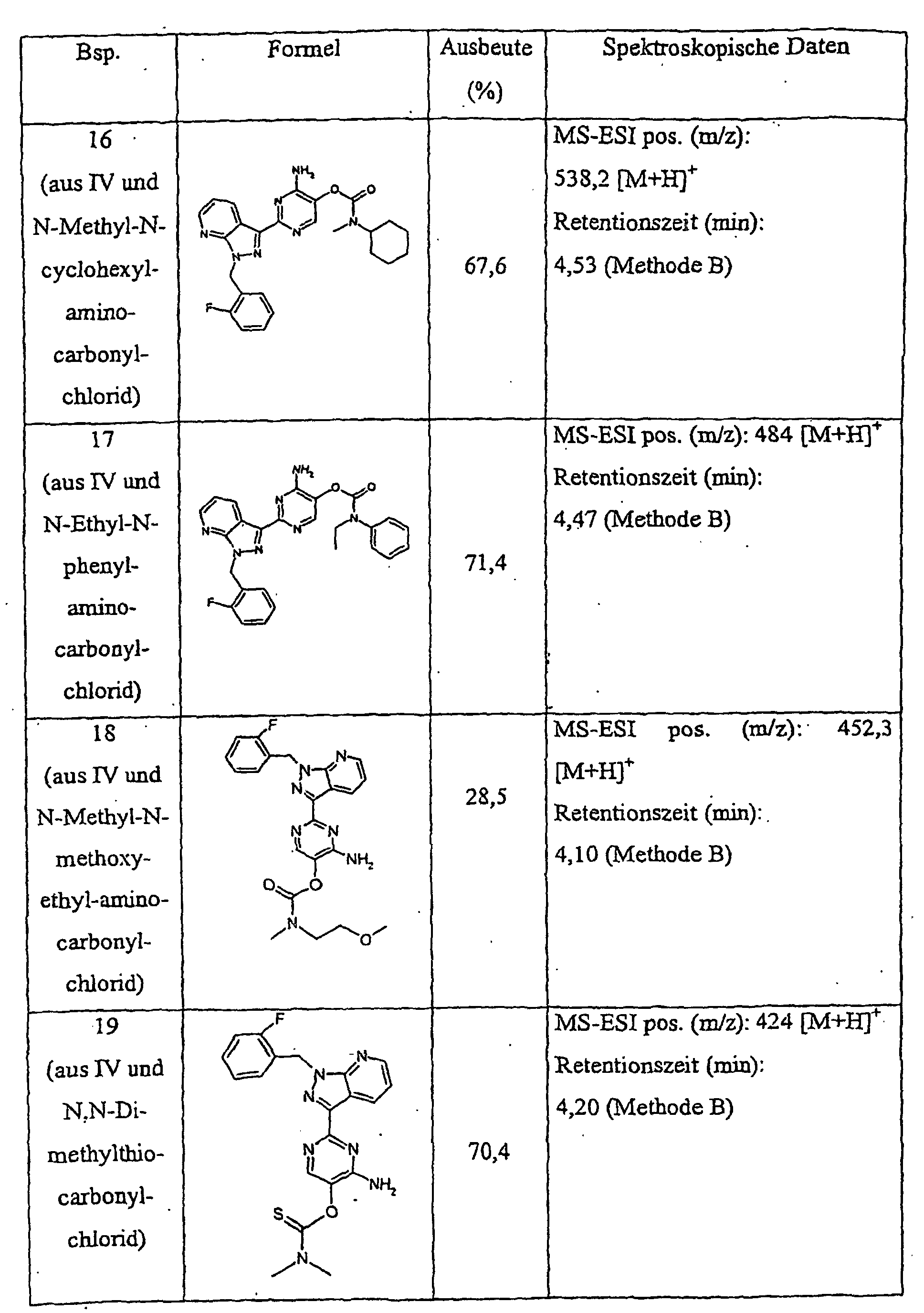

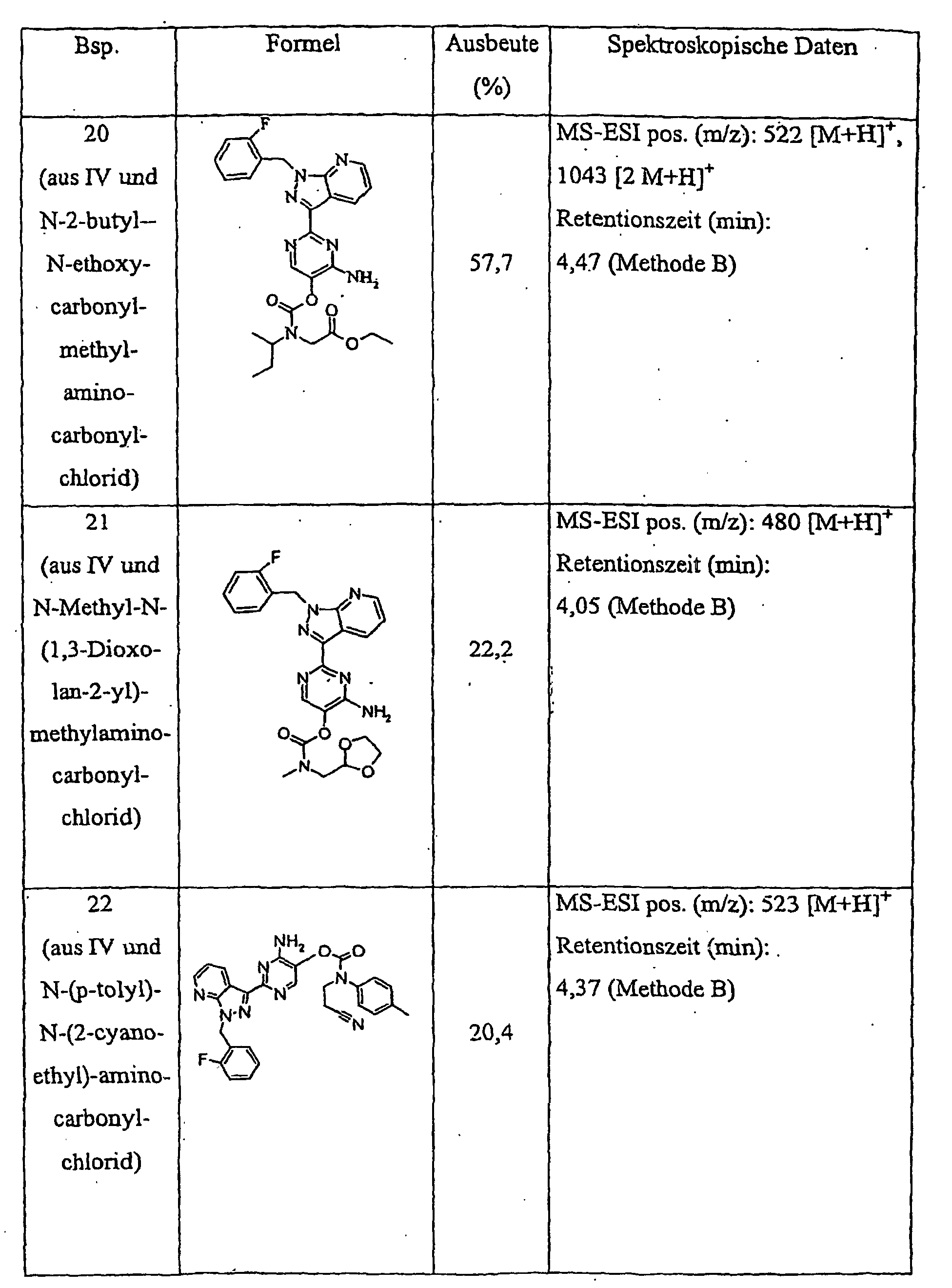

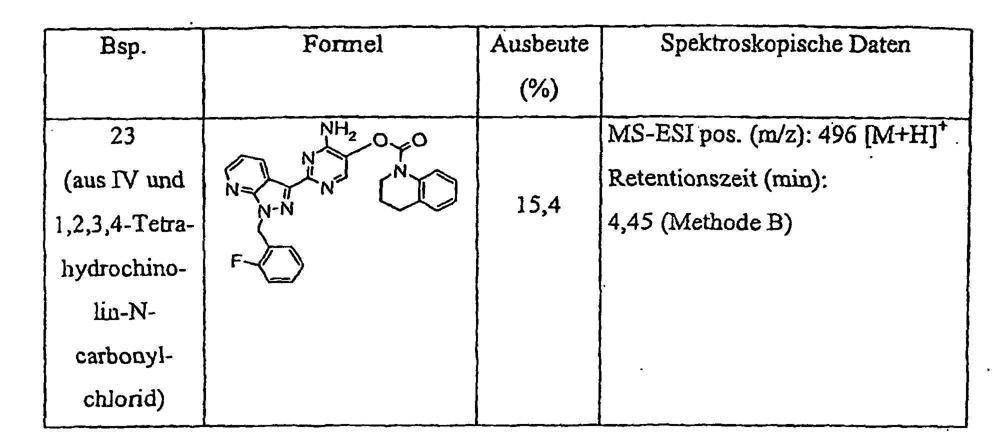

### 24. 4-Amino-2-[1-(2-fluorobenzyl)-1H-pyrazolo[3,4-b]pyridin-3-yl]-5-pyrimidinyl 2-methoxyethylcarbamat

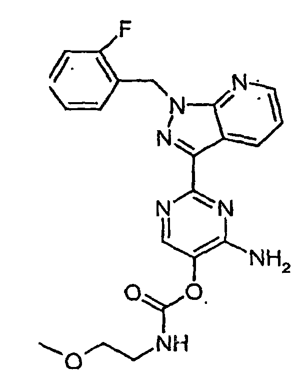

160.3 mg (0.29 mmol) 4-Amino-2-[1-(2-fluorobenzyl)-1H-pyrazolo[3,4-b]pyridin-3-yl]-5-pyrimidinyl-4-methoxybenzyl(2-methoxyethyl)carbamat aus Bsp. IX werden in 3 ml Trifluoressigsäure gelöst und 6h bei 60°C gerührt. Zur Aufarbeitung gießt man die Reaktionslösung auf Eiswasser, neutralisiert vorsichtig mit Natriumhydrogencarbonat, extrahiert 3x mit Dichlormethan, trocknet die org. Phase über Natriumsulfat und engt ein. Der Rückstand wird über Kieselgel mit Dichlormethan/Methanol (30:1) chromatographiert.
- Ausbeute:: 28.3 g (22.5%)
- Rf-Wert:: 0.88 (Dichlormethan/Methanol 10:1)
- ¹H-NMR:: (200 MHz, CDCl3): δ = 3.39 (s, 3H, OCH3), 3,43-3,58 (m, 4H, CH2CH2), 5,15-5,28 (br. s, 2H, NH2), 5,50-5,63 (m, 1H, NH), 5,93 (s, 2H, CH2), 6,86-7,30 (m, 5H, Ar-H), 8.37 (s, 1H, Ar-H); 8.59 (dd, 1H, Ar-H), 8.92 (dd, 1H, Ar-H).
- MS:: ESI pos.: *m*/*z* = 438,2 [M+H]+

### 25. 4-Amino-2-[1-(2-fluorobenzyl)-1H-pyrazolo[3,4-b]pyridin-3-yl]-5-pyrimidinyl-1-piperazinecarboxylat

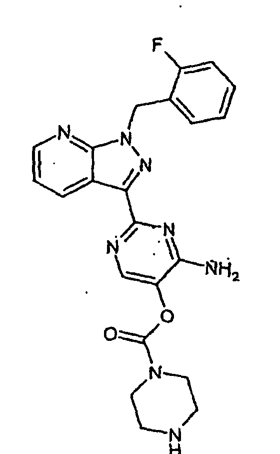

214.2 mg (0.390 mmol) 1-{4-Amino-2-[1-(2-fluorobenzyl)-1H-pyrazolo[3,4-b]pyridin-3-yl]-5-pyrimidinyl}-4-tert-butyl-1,4-piperazindicarboxylat aus Beispiel 4 werden in 5 ml Dichlormethan gelöst und mit 5 ml Trifluoressigsäure versetzt. Man lässt 1h bei Raumtemperatur rühren. Zur Aufarbeitung neutralisiert man mit 1N Natriumhydroxid-Lösung und extrahiert dreimal mit Dichlormethan. Die organische Phase wird über Natriumsulfat getrocknet und am Rotationsverdampfer eingeengt.
- Ausbeute:: 123.1 mg (64.0 %)
- Rf-Wert:: 0.16 (Dichlormethan/Methanol 10:1)
- ¹H-NMR:: (200 MHz, D₆-DMSO). δ = 2.67-2.81. (br.s, 4H, Piperazin-H), 3.21-3.41 (br.s, 2H, Piperazin-H, fällt zusammen mit H₂O-Signal), 3.42-3.59 (br.s, 2H, Piperazin-H), 5.81 (s, 2H, CH₂), 7.08-7.41 (m, 7H, Ar-H und NH₂), 8.10 (s, 1H, Pyrimidin-H), 8.63 (dd, 1H, Pyridin-H), 8.93 (dd, 1H, Pyridin-H)
- MS:: (ESI pos.), *m*/*z* = 449.2 ([M+H]⁺)

### 26. 4- {[(Diethylamino)carbonyl]amino}-2-[1-(2-fluorobenzyl)-1H-pyrazolo[3,4-b]pyridin-3-yl]-5-pyrimidinyldiethylcarbamat

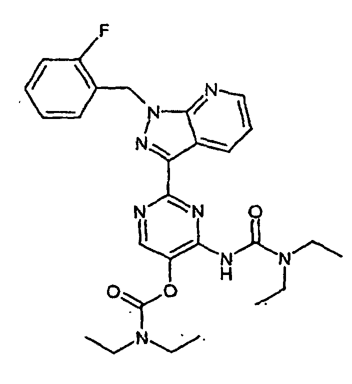

100 mg (0.297 mmol)) 4-Amino-2-[1-(2-fluorobenzyl)-1H-pyrazolo[3,4-b]pyridin-3-yl]-5-pyrimidinol aus Beispiel IV werden in 5 ml wasserfreiem THF suspendiert und mit 7.51 mg (0.297 mmol) Natriumhydrid (95%ig) versetzt. Man lässt 30 min bei Raumtemperatur rühren. Nun gibt man 44:35 mg (0.327 mmol) N,N-Diethylcarbamoylchlorid zu und lässt über Nacht bei Raumtemperatur rühren. Man gibt vorsichtig Wasser zu und extrahiert dreimal mit Ethylacetat. Die organische Phase wird über Natriumsulfat getrocknet und am Rotationsverdampfer eingeengt. Der Rückstand wird über Kieselgel mit Dichlormethan/Methanol 100:1 und anschließend über präparative HPLC (Säule: Kromasil 100 C 18 5 µm 250x20 mm Nr. 101132R, Fluss: 25 ml/min, Temp. 50°C, Wasser/Acetonitril 50/50) chromatographiert.
- Ausbeute:: 29.9 g (18.8 %)
- Rf-Wert:: 0.88 (Dichlormethan/Methanol 10:1)
- ¹H-NMR:: (400 MHz, D₆-DMSO), δ = 1.07-1.19 (m, 12H, CH₃CH₂), 3.26-3.41 (m, 8H, CH₃CH₂), 5.85 (s, 2H, CH₂), 7.11-7.44 (m, 5H, Ar-H), 8.52 (s, 1H, Pyrimidin-H), 8.68 (dd, 1H, Pyridin-H), 9.02 (dd, 1H, Pyridin-H), 9.39 (br. s, 1H, NH)

### 27. 4-Amino-2-[1-(2-fluorobenzyl)-1H-pyrazolo[3,4-b]pyridin-3-yl]-5-pyrimidinyl- methylcarbamat

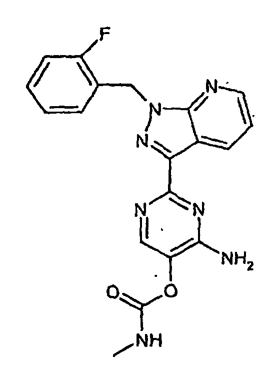

562.5 mg (1.04 mmol) 4-Amino-2-[1-(2-fluorobenzyl)-1H-pyrazolo[3,4-b]pyridin-3-yl]-5-pyrimidinyl-3,4-dimethoxybenzyl(methyl)carbamat aus Beispiel VI werden in 10 ml Trifluoressigsäure gelöst und 4h bei 60°C gerührt. Zur Aufarbeitung gießt man die Reaktionslösung auf Eiswasser, neutralisiert vorsichtig mit Natriumhydrogencarbonat, extrahiert dreimal mit Dichlormethan, trocknet die organische Phase über Natriumsulfat und engt ein. Der Rückstand wird über Kieselgel mit Dichlormethan/Methanol (zunächst 100:1, dann 30:1) chromatographiert.
- Ausbeute:: 123.8 g (30.4 % über 2 Stufen)
- MS(MALDI pos.):: *mlz* = 394.18 ([M+H]⁺, 416.15 ([M+Na]⁺)
- Rf-Wert:: 0.67 (Dichlormethan/Methanol 10:1)
- ¹H-NMR:: (200 MHz, D₆-DMSO). Das Spektrum weißt E/Z-Rotamerie der Carbamat-Gruppe auf. Im folgenden werden nur die Signale des Hauptrotamers beschrieben: δ = 2.70 (d, 3H, CH₃), 5.81 (s, 2H, CH₂), 7.07-7.41 (m, 7H, Ar-H und NH₂), 7.62 (br. q, 1H, NH), 8.11 (s, 1H, Pyrimidin-H], 8.62 (dd, 1H, Pyridin-H), 8.93 (dd, 1H, Pyridin-H)

### 28. 4-Amino-2-[1-(2-fluorobenzyl)-1H-pyrazolo[3,4-b]pyridin-3-yl]-5-pyrimidinyl- allyl(cyclopentyl)carbamat

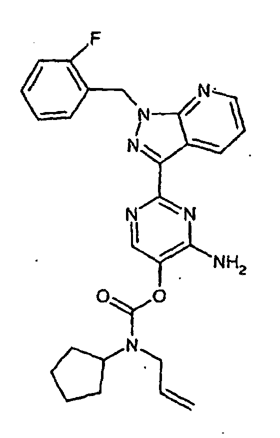

100 mg (0.297 mmol) 4-Ammo-2-[1-(2-fluorobenzyl)-1H-pyrazolo[3,4-b]pyridin-3-yl]-5-pyrimidinol aus Beispiel IV werden in 15 ml wasserfreiem Pyridin suspendiert und auf 110°C erhitzt. Dazu gibt man portionsweise 67.0 mg (0.357 mmol) Allyl(cyclopentyl)carbamoylchlorid aus Beispiel VII und lässt 2h bei 110°C rühren. Zur Aufarbeitung engt man die Reaktionslösung ein, nimmt den Rückstand in Dichlormethan auf, extrahiert einmal mit gesättigter Ammoniumchlorid-Lösung und einmal mit gesättigter NaCl-Lösung, trocknet die organische Phase über Na₂SO₄ und engt am Rotationsverdampfer ein. Der Rückstand wird über Kieselgel mit Dichlormethan/Methanol 100:1 chromatographiert.
- Ausbeute:: 105 mg (72 %)
- Rf-Wert:: 0.92 (Dichlormethan/Methanol 10:1)
- ¹H-NMR:: (200 MHz, D₆-DMSO), δ = 1.37-1.98 (br. m, 8H, Cyclopentyl-CH₂), 3.80-4.05 (br. m, 2H, Allyl-CH₂N), 4.22-4.53 (br. m, 1H, Cyclopentyl-CHN),
5.07-5.29 (br. m, 2H, olefinische Allyl-CH₂), 5.80-6.10 (br. m, 1H, olefinische Allyl-CH), überlagert von 5.81 (s, 2H, CH₂), 7.04-7.41 (m, 7H, Ar-H und NH₂), 8.08(s, 1H, Pyrimidin-H), 8.64(dd, 1H, Pyridin-H), 8.92 (dd, 1H, Pyzidin-H)
- MS:: (ESI pos.), *m*/*z* = 488 ([M+H]⁺), 975 ([2M+H]⁺)

### 29. N-[({4-Amino-2-[1-(2-fluorobenzyl)-1H-pyrazolo[3,4-b]pyridin-3-yl]-5-pyrimidinyl}oxy)carbonyl]-N-(sec-butyl)glycin

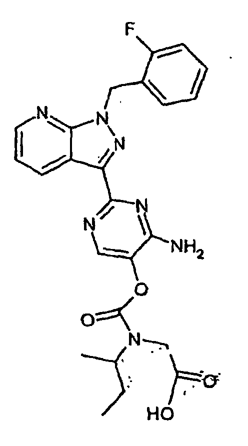

73.5 mg (0.141 mmol) Ethyl-N-[({4-amino-2-[1-(2-fluorobenzyl)-1H-pyrazolo[3,4-b]pyridin-3-yl]-5-pyrimidinyl}oxy)carbonyl]-N-(sec-butyl)glycinate aus Beispiel 20 werden in 3 ml THF/ Wasser/ Methanol 1:1:1 gelöst. Dazu gibt man 5.1 mg (0.12 mmol) Lithiumhydroxid-monohydrat und lässt 1h bei RT rühren. Zur Aufarbeitung extrahiert man 3x mit je 10 ml CH₂Cl₂ und 1x mit gesättigter NaCl-Lsg., trocknet über Na₂SO₄ und engt am Rotationsverdampfer zur Trockne ein.
Der Rückstand wird über präparative HPLC (Säule: Cromsil 120 ODS, C-18, 10 µm, 250X30 mm, Fluss 50 ml/min, Raumtemp., Gradient: Wasser Acetonitril bei 0 min: 90:10, bei 28 min 5:95) gereinigt.
- Rf-Wert:: 0,25 (CH₂Cl₂/ CH₃OH 10:1)
- HPLC-Retentionszeit:: 3.55 (Methode F)
- MS-ESI pos.:: (m/z): 494.3 [M+H]⁺, 987.3 [2M+H]⁺

## Patentansprüche

1. Verbindungen der allgemeinen Formel (I) 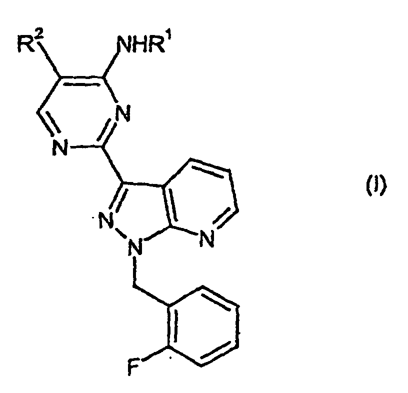 worin
R¹ für Wasserstoff oder einen Di-C₁₋₆-alkylaminocarbonylrest steht,
R² für einen Rest der Formel -O-CX-NR³R⁴ steht,
wobei
X für O oder S steht;
R³ und R⁴ gleich oder voneinander verschieden sein können und für einen Rest aus der Gruppe, bestehend aus H, C₁₋₆-Alkyl, das gegebenenfalls einen CN- oder einen Halogensubstituenten trägt, C₁₋₆-Alkoxy-C₁₋₆-alkyl, Hydroxy-C₁₋₆-alkyl, C₂₋₆-Alkenyl, C₁₋₆-Alkylcarbonyloxy-C₁₋₆-alkyl, C₁₋₆-Alkoxycarbonyl-C₁₋₆-alkyl, Hydroxycarbonyl-C₁₋₆-alkyl, Phenyl, p-Tolyl, einem über einen C₁₋₆-Alkandiylrest an das Stickstoffatom gebundenen gesättigten fünf- bis siebengliedrigen Heterocyclus mit bis zu 2 Sauserstoffatomen oder C₃₋₈-Cycloalkyl, steht, wobei R³ und R⁴ nicht gleichzeitig H sein können;
oder
R³ und R⁴ zusammen mit dem Stickstoffatom, an das sie gebunden sind, einen fünf- oder sechsgliedrigen gesättigten Heterocyclus bilden, der gegebenenfalls ein weiteres Heteroatom aus der Gruppe N, O, S enthalten und/oder gegebenenfalls einen Alkylcarbonyl- oder Alkoxycarbonyl-Substitnenten kann;
sowie Salze, Isomere und Hydrate davon.

2. Verbindungen nach Anspruch 1,
worin
R¹ für Wasserstoff oder einen Diethylaminocarbonylrest steht,
R² für einen Rest der Formel -O-CX-NR³R⁴ steht,
wobei
X für O oder S steht;
R³ und R⁴ gleich oder voneinander verschieden sein können und für einen Rest aus der Gruppe, bestehend aus H, Methyl, Ethyl, iso-Propyl, Butan-2-yl, Methoxyethyl, 2-Methoxy-1-methylethyl, 1-Cyano-1-methylethyl, 2-Cyanoethyl, 2-Chlorethyl, Ethoxycarbonylmethyl, Hydroxycarbonylmethyl 2-Propenyl, Phenyl, p-Tolyl, 1,3-Dioxolan-2-methyl, Cyclohexyl oder Cyclopentyl, steht, wobei R³ und R⁴ nicht gleichzeitig H sein können;
oder
R³ und R⁴ zusammen mit dem Stickstoffatom, an das sie gebunden sind, einen fünf- oder sechsgliedrigen gesättigten Heterocyclus bilden, der gegebenenfalls ein weiteres Heteroatom aus der Gruppe N, O enthalten und/oder gegebenenfalls einen Substituenten aus der Gruppe Methylcarbonyl, Ethoxycarbonyl oder t-Butoxycarbonyl, tragen kann, oder zusammen für 1,2,3,4-Tetrahydrochinolin-N-yl stehen;
sowie Salze, Isomere und Hydrate davon.

3. Verfahren zur Herstellung von Verbindungen der Formel I, umfassend die Umsetzung der Verbindung der Formel (II) 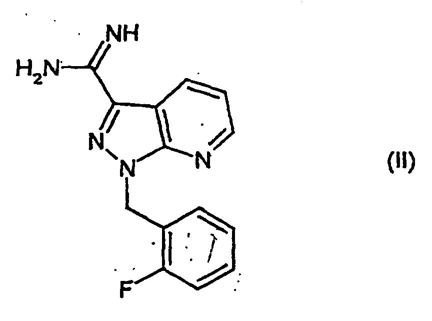 mit der Verbindung der Formel (III) 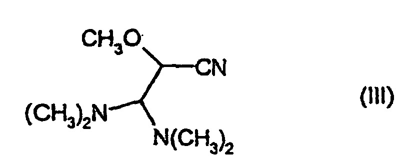 in einem organischen Lösungsmittel in Gegenwart einer Base unter Erhitzen und anschließender Überführung der Ethergruppe in die freie Hydroxygruppe zu Verbindungen der Formel (IV) 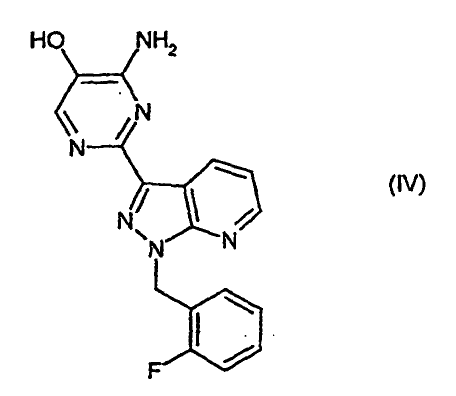 sowie die anschließende Umsetzung mit Verbindungen der Formel X-CO-NR³R⁴,
worin
X für einen Halogenrest oder Alkoxyrest steht,
R³ und R⁴ die in Anspruch 1 angegebene Bedeutung haben;
in einem organischen Lösungsmittel gegebenenfalls in Gegenwart einer Base sowie gegebenenfalls unter anschließender Entfernung von Schutzgruppen zu Verbindungen der Formel (I).

4. Verbindungen der allgemeinen Formel (I) gemäß Anspruch 1 zur Behandlung von Krankheiten.

5. Arzneimittel enthaltend mindestens eine Verbindung der allgemeinen Formel (I) gemäß Anspruch 1.

6. Vezfahren zur Herstellung von Arzneimitteln, **dadurch gekennzeichnet, dass** man mindestens eine Verbindung der Formel (I) gemäß Anspruch 1, gegebenenfalls mit üblichen Hilfs- und Zusatzstoffen in eine geeignete Applikationsform überführt.

7. Arzneimittel enthaltend mindestens eine Verbindung der allgemeinen Formel (I) gemäß Anspruch 1 in Kombination mit organischen Nitraten oder NO-Donatoren.

8. Arzneimittel enthaltend mindestens eine Verbindung der allgemeinen Formel (I) gemäß Anspruch 1 in Kombination mit Verbindungen, die den Abbau von cyclischen Guanosinmonophosphat (cGMP) inhibieren.

9. Verwendung von Verbindungen der allgemeinen Formel (I) gemäß Anspruch 1 bei der Herstellung von Arzneimitteln zur Behandlung von Fterz-Kreislauf-Erkrankungen.

10. Verwendung von Verbindungen der allgemeinen Formel (I) gemäß Anspruch 1 bei der Herstellung von Arzneimitteln zur Behandlung von Hypertonie.

11. Verwendung von Verbindungen der allgemeinen Formel (I) gemäß Anspruch 1 bei der Herstellung von Arzneimitteln zur Behandlung von thromboembo-Lischen Erkrankungen und Ischämien.

12. Verwendung von Verbindungen der allgemeinen Formel (I) gemäß Anspruch 1 bei der Herstellung von Arzneimitteln zur Behandlung von sexueller Dysfunktion.

13. Verwendung von Verbindungen der allgemeinen Formel (I) gemäß Anspruch 1 bei der Herstellung von Arzneimitteln mit antiinflammatorischen Eigenschaften.

14. Verwendung von Verbindungen der allgemeinen Formel (I) gemäß Anspruch 1 bei der Herstellung von Arzneimitteln zur Behandlung von Erkrankungen des Zentralnervensystems.

15. Verwendung gemäß einem der Ansprüche 9 bis 14, wobei die Verbindungen der allgemeinen Formel gemäß Anspruch 1 in Kombination mit organischen Nitraten oder NO-Donatoren oder in Kombination mit Verbindungen, die den Abbau von cyclischen Guanosinmonophosphat (cGMP) inhibieren, eingesetzt werden.

## Claims

1. Compounds of the formula (I) 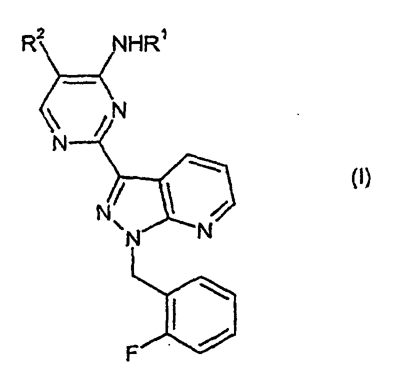 in which
R¹ is hydrogen or a di-C₁₋₆-alkylaminocarbonyl radical,
R² is a radical of the formula -O-CX-NR³R⁴,
where
X is 0 or S;
R³ and R⁴ may be identical or different and is a radical from the group consisting of H, C₁₋₆-alkyl which optionally has a CN or a halogen substituent, C₁₋₆-alkoxy-C₁₋₆-alkyl, hydroxy-C₁₋₆-alkyl, C₂₋₆-alkenyl, C₁₋₆-alkylcarbonyloxy-C₁₋₆-alkyl, C₁₋₆-alkoxycarbonyl-C₁₋₆-alkyl, hydroxycarbonyl-C₁₋₆-alkyl, phenyl, p-tolyl, a saturated five- to seven-membered heterocycle which is linked via a C₁₋₆-alkanediyl radical to the nitrogen atom and has up to 2 oxygen atoms, or C₃₋₈-cycloalkyl, where R³ and R⁴ cannot simultaneously be H;
or
R³ and R⁴ together with the nitrogen atom to which they are bonded form a five- or six-membered saturated heterocycle which may optionally contain a further heteroatom from the group of N, O, S and/or optionally an alkylcarbonyl or alkoxycarbonyl substituent;
and salts, isomers and hydrates thereof.

2. Compounds according to Claim 1,
in which
R¹ is hydrogen or a diethylaminocarbonyl radical,
R² is a radical of the formula -O-CX-NR³R⁴,
where
X is O or S;
R³ and R⁴ may be identical or different and is a radical from the group consisting of H, methyl, ethyl, isopropyl, butan-2-yl, methoxyethyl, 2-methoxy-1-methylethyl, 1-cyano-2-methylethyl, 2-cyanoethyl, 2-chloroethyl, ethoxycarbonylmethyl, hydroxycarbonylmethyl 2-propenyl, phenyl, p-tolyl, 1,3-dioxolan-2-methyl, cyclohexyl or cyclopentyl, where R³ and R⁴ cannot simultaneously be H;
or
R³ and R⁴ together with the nitrogen atom to which they are bonded form a five- or six-membered saturated heterocycle which may optionally contain a further heteroatom from the group of N, O and/or may optionally have a substituent from the group of methylcarbonyl, ethoxycarbonyl or t-butoxycarbonyl, or together are 1,2,3,4-tetrahydroquinolin-N-yl;
and salts, isomers and hydrates thereof.

3. Process for preparing compounds of the formula 1, comprising reaction of the compound of the formula (II) 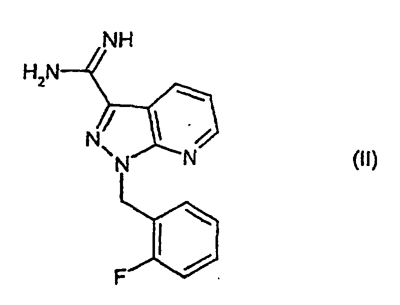 with the compound of the formula (III) 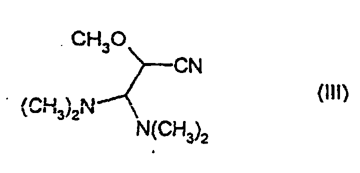 in an organic solvent in the presence of a base with heating and subsequent conversion of the ether group into the free hydroxyl group to give compounds of the formula (IV) 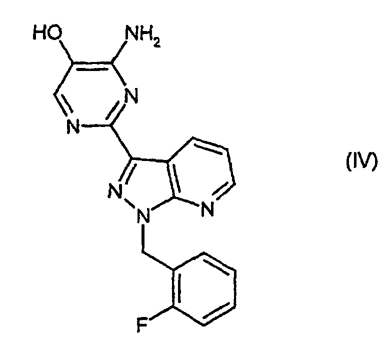 and subsequent reaction with compounds of the formula X-CO-NR³R⁴
in which
X is a halogen radical or alkoxy radical,
R³ and R⁴ have the meaning indicated in Claim 1; in an organic solvent, where appropriate in the
presence of a base, and where appropriate with subsequent removal of protective groups to give compounds of the formula (I).

4. Compounds of the formula (I) according to Claim 1 for the treatment of diseases.

5. Medicament comprising at least one compound of the formula (I) according to Claim 1.

6. Process for producing medicaments, **characterized in that** at least one compound of the formula (I) according to Claim 1 is converted, where appropriate with conventional excipients and additives, into a suitable administration form.

7. Medicament comprising at least one compound of the formula (I) according to Claim 1 in combination with organic nitrates or NO donors.

8. Medicament comprising at least one compound of the formula (I) according to Claim 1 in combination with compounds which inhibit the breakdown of cyclic guanosine monophosphate (cGMP).

9. Use of compounds of the formula (I) according to Claim 1 in the production of medicaments for the treatment of cardiovascular disorders.

10. Use of compounds of the formula (I) according to Claim 1 in the production of medicaments for the treatment of hypertension.

11. Use of compounds of the formula (I) according to claim 1 in the production of medicaments for the treatment of thromboembolic disorders and ischemias.

12. Use of compounds of the formula (I) according to claim 1 in the production of medicaments for the treatment of sexual dysfunction.

13. Use of compounds of the formula (I) according to Claim 1 in the production of medicaments having anti-inflammatory properties.

14. Use of compounds of the formula (I) according to Claim 1 in the production of medicaments for the treatment of disorders of the central nervous system.

15. Use according to any of Claims 9 to 14, where the compounds of the formula according to Claim 1 are employed in combination with organic nitrates or NO donors or in combination with compounds which inhibit the breakdown of cyclic guanosine monophosphate (cGMP).

## Revendications

1. Composés de formule générale (I) 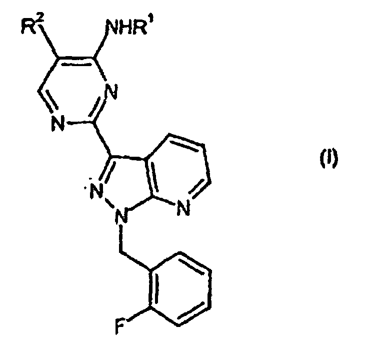 dans laquelle
R¹ représente l'hydrogène ou un reste di(alkyle en C₁ à C₆)aminocarbonyle,
R² représente un reste de formule -O-CX-NR³R⁴,
où
X représente O ou S ;
R³ et R⁴ peuvent être identiques ou différents et peuvent représenter un reste du groupe constitué de H, alkyle en C₁ à C₆ qui porte éventuellement un substituant CN- ou un substituant halogéno, (alkoxy en C₁ à C₆) (alkyle en C₁ à C₆), hydroxy (alkyle en C₁ à C₆), alcényle en C₂ à C₆, (alkyle en C₁ à C₆) carbonyloxy (alkyle en C₁ à C₆), (alkoxy en C₁ à C₆) carbonyl (alkyle en C₁ à C₆), hydroxycarbonyl (alkyle en C₁ à C₆), phényle, p-tolyle, un hétérocycle pentagonal à heptagonal saturé ayant jusqu'à 2 atomes d'oxygène, lié à l'atome d'azote par l'intermédiaire d'un reste alcanediyle en C₁ à C₆, ou bien cycloalkyle en C₃ à C₈, R³ et R⁴ ne pouvant pas représenter en même temps H ;
ou bien
R³ et R⁴ forment conjointement avec l'atome d'azote auquel ils sont liés un hétérocycle saturé pentagonal ou hexagonal qui peut éventuellement contenir un autre hétéroatome du groupe N, O, S
et/ou peut éventuellement porter un substituant alkylcarbonyle ou alkoxycarbonyle ;
ainsi que leurs sels, leurs isomères et leurs hydrates.

2. Composés suivant la revendication 1,
dans lesquels
R¹ représente l'hydrogène ou un reste diéthylaminocarbonyle,
R² représente un reste de formule -O-CX-NR³R⁴,
où
X représente O ou S ;
R³ et R⁴ peuvent être identiques ou différents et peuvent représenter un reste du groupe constitué de H, méthyle, éthyle, isopropyle, butane-2-yle, méthoxyéthyle, 2-méthoxy-1-méthyléthyle, 1-cyano-1-méthyléthyle, 2-cyanéthyle, 2-chloréthyle, éthoxycarbonylméthyle, hydroxycarbonylméthyle, 2-propényle, phényle, p-tolyle, 1,3-dioxolanne-2-méthyle, cyclohexyle ou cyclopentyle, R³ et R⁴ ne pouvant pas représenter en même temps H ;
ou bien
R³ et R⁴ forment conjointement avec l'atome d'azote auquel ils sont liés un hétérocycle saturé pentagonal ou hexagonal qui peut contenir éventuellement un autre hétéroatome du groupe N, O et/ou qui peut éventuellement porter un substituant du groupe méthylcarbonyle, éthoxycarbonyle ou tertiobutoxy-carbonyle, ou forment ensemble un reste 1,2,3,4-tétrahydroquinoléine-N-yle ;
ainsi que leurs sels, leurs isomères et leurs hydrates.

3. Procédé de production de composés de formule (I) comprenant la réaction à chaud du composé de formule (II) 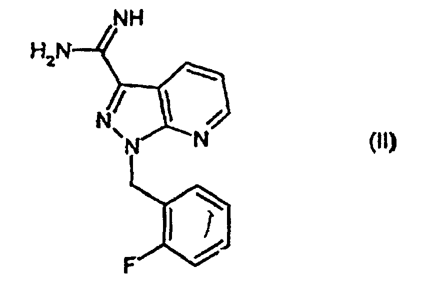 avec le composé de formule (III) 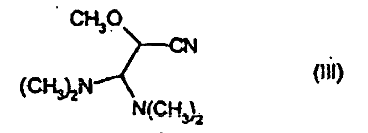 dans un solvant organique en présence d'une base, suivie de la transformation du groupe éther en le groupe hydroxy libre pour obtenir des composés de formule (IV) 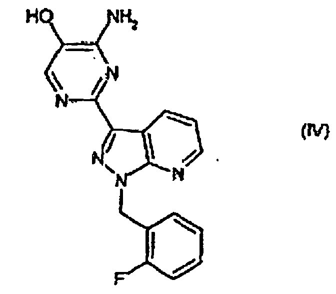 ainsi que la réaction subséquente avec des composés de formule X-CO-NR³R⁴, dans laquelle
X représente un reste halogénure ou un reste alkoxy,
R³ et R⁴ ont la définition indiquée dans la revendication 1 ;
dans un solvant organique, éventuellement en présence d'une base ainsi que, le cas échéant, avec élimination subséquente de groupes protecteurs pour obtenir des composés de formule (I).

4. Composés de formule générale (I) suivant la revendication 1, destinés au traitement de maladies.

5. Médicament contenant au moins un composé de formule (I) suivant la revendication 1.

6. Procédé de préparation de médicaments, **caractérisé en ce qu'**on fait prendre une forme d'administration convenable à au moins un composé de formule (I) suivant la revendication 1, éventuellement avec des substances auxiliaires et des additifs usuels.

7. Médicament contenant au moins un composé de formule générale (I) suivant la revendication 1 en association avec des nitrates organiques ou des donneurs de NO.

8. Médicament contenant au moins un composé de formule générale (I) suivant la revendication 1 en association avec des composés qui inhibent la dégradation du monophosphate de guanosine cyclique (GMPc).

9. Utilisation de composés de formule générale (I) suivant la revendication 1, pour la préparation de médicaments destinés au traitement de maladies cardiovasculaires.

10. Utilisation de composés de formule générale (I) suivant la revendication 1, pour la préparation de médicaments destinés au traitement de l'hypertonie.

11. Utilisation de composés de formule générale (I) suivant la revendication 1, pour la préparation de médicaments destinés au traitement de maladies thromboemboliques et d'ischémies.

12. Utilisation de composés de formule générale (I) suivant la revendication 1, pour la préparation de médicaments destinés au traitement du dysfonctionnement sexuel.

13. Utilisation de composés de formule générale (I) suivant la revendication 1, pour la préparation de médicaments doués de propriétés anti-inflammatoires.

14. Utilisation de composés de formule générale (I) suivant la revendication 1, pour la préparation de médicaments destinés au traitement de maladies du système nerveux central.

15. Utilisation suivant l'une des revendications 9 à 14, dans laquelle les composés de formule générale suivant la revendication 1 sont utilisés en association avec des nitrates organiques ou des donneurs de NO ou en association avec des composés qui inhibent la dégradation du monophosphate de guanosine cyclique (GMPc).
